# EUROPEAN PATENT SPECIFICATION

(11) **EP 2 395 982 B1**
(45) Date of publication and mention of the grant of the patent: **22.10.2014**
(21) Application number: 09805977.7
(22) Date of filing: 21.12.2009
(51) Int. Cl.: A61K 9/00, A61M 5/142, A61K 31/7036

(54) **AN IMPLANTABLE COMPOSITE COMPRISING A TACKY BIOCOMPATIBLE POLYMER FILM AND A RELEASABLE BIOACTIVE AGENT**
IMPLANTIERBARES KOMPOSITMATERIAL ENTHALTEND EINEN KLEBRIGEN BIOKOMPATIBLEN POLYMERFILM UND EIN FREISETZBARES BIOLOGISCH WIRKSAMES MITTEL
COMPOSITES IMPLANTABLES COMPRENANT UN FILM DE POLYMÈRE ADHÉSIF BIOCOMPATIBLE ET UN AGENT BIOLOGIQUEMENT ACTIF LIBÉRABLES

(30) Priority: 23.12.2008 US 140492 P; 23.12.2008 US 140486 P; 23.12.2008 US 140449 P; 23.12.2008 US 140468 P; 23.12.2008 US 140476 P; 23.12.2008 US 140417 P
(43) Date of publication of application: 21.12.2011
(73) Proprietor: Surmodics Pharmaceuticals, Inc., Birmingham, AL 35211 (US)
(72) Inventor: TIPTON, Arthur, J., Homewood AL 35209 (US); BURTON, Kevin, W., Hoover AL 35226 (US); TICE, Thomas, R., Indian Springs AL 35124 (US); BOWMAN, Howard, Birmingham AL 35242 (US); BIGGS, Danielle, Memphis TN 38132 (US); MARKLAND, Peter, Birmingham AL 35244 (US)
(74) Representative: Leissler-Gerstl, Gabriele
(86) International application number: PCT/US2009/069024
(87) International publication number: WO 2010/075298

(56) References cited:
- EP-A2- 0 306 212
- EP-A2- 2 050 474
- WO-A1-2006/124021
- US-A1- 2003 185 872

## Description

### BACKGROUND

In medicine, certain disorders and conditions require medical implants. Medical implants are often used to replace a damaged biological tissue or fluid, augment or enhance a biological process, enhance the healing of a surgical site, deliver a drug to a localized site within a subject, or perform another biological or structural role. Implants can even be necessary to keep a patient alive. Unfortunately, problems can arise during an implant surgery, or after a patient has received the medical implant. In some instances, the implant can impair healing of the surgical site. For example, the surface of the implant can recruit cellular debris and other biological material that can become infected with bacteria, fungus, or other infectious agents. The subject's immune system can also recognize the implant as a foreign body, and attempt to fight the implant using natural defenses. This often lowers the strength of the subject's immune system and can lead to further serious problems, such as periprosthetic infections, or other infections at or near the surgical implant site.

US2003185872, EP0306212, WO2006124021 and EP2050474 disclose implants comprising active principles which may include adhesive layers, adherent coatings microspheres.

Accordingly, it can also be desirable to deliver a bioactive agent at or near the tissue adjacent the implant site. Such a bioactive agent can help prevent at least some of the aforementioned problems associated with implants, or enhance the function of the implant itself. Unfortunately, configuring each implant to be capable of locally delivering a bioactive agent is not always possible or practical. For example, regulations for the manufacture of drug products differ significantly from the regulations for the manufacture of medical devices.

As such, a need exists for composites and compositions that can be applied to an implant or implanted into a subject that effectively provide a bioactive agent at or near tissue adjacent the implant site. These needs and other needs are satisfied by the present invention.

### SUMMARY

Described herein are implantable composites and compositions, kits comprising the implantable composites and compositions, and implant devices comprising the implantable composites and compositions. In one aspect, disclosed are point of use applications, wherein a bioactive agent is applied to a medical device close to the time of use, which allows for the separate and more rapid development of the bioactive agent and the implant device, such that the quality or efficacy of the final implant device is not unduly compromised (see definition according to claim 1).

Implantable film-based composites, implantable elastic band composites, implantable flexible body composites, implantable suction cup composites, terpolymer compositions, spray coating compositions, methods for using the composites and compositions, and implant devices comprising the composites and compositions are each disclosed and described herein.

The advantages of the invention will be set forth in part in the description which follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE DRAWINGS

Figure 1A is a drawing of an exemplary implantable film-based composite.
Figure 1B is a drawing of another exemplary implantable film-based composite.
Figure 2 is a drawing of an exemplary kit comprising a plurality of implantable film-based composites.
Figure 3 is a drawing of an exemplary implant device comprising an implantable film-based composite.
Figure 4 is a drawing of an exemplary implantable elastic band composite.
Figure 5 is a drawing of an exemplary implant device comprising an implantable elastic band composite.
Figure 6A is a drawing of an exemplary implantable flexible body composite wherein the first portion and second portion are not connected.
Figure 6B is a drawing of an exemplary implantable flexible body composite wherein the first portion and second portion are connected.
Figure 7 is a drawing of an exemplary implant device comprising an implantable flexible body composite secured thereto.
Figure 8 is a drawing of an exemplary implantable suction cup composite.
Figure 9 is a drawing of an exemplary implant device comprising an implantable suction cup composite adhered to the device.

### DETAILED DESCRIPTION

In this specification and in the claims that follow, reference will be made to a number of terms that shall be defined to have the following meanings:
Throughout this specification, unless the context requires otherwise, the word "comprise," or variations such as "comprises" or "comprising," will be understood to imply the inclusion of a stated integer or step or group of integers or steps but not the exclusion of any other integer or step or group of integers or steps.

It must be noted that, as used in the specification and the appended claims, the singular forms "a," "an" and "the" include plural referents unless the context clearly dictates otherwise. Thus, for example, reference to "a bioactive agent" includes mixtures of two or more such agents, and the like.

"Optional" or "optionally" means that the subsequently described event or circumstance can or cannot occur, and that the description includes instances where the event or circumstance occurs and instances where it does not.

Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

The term "biocompatible" refers a substance that is substantially non-toxic to a subject.

"Biodegradable" is generally referred to herein as a material that will erode to soluble species or that will degrade under physiologic conditions to smaller units or chemical species that are, themselves, non-toxic (biocompatible) to the subject and capable of being metabolized, eliminated, or excreted by the subject.

A "bioactive agent" refers to an agent that has biological activity. The biological agent can be used to treat, diagnose, cure, mitigate, prevent (*i.e.,* prophylactically), ameliorate, modulate, or have an otherwise favorable effect on a disease, disorder, infection, and the like. A "releasable bioactive agent" is one that can be released from a disclosed composite or composition. Bioactive agents also include those substances which affect the structure or function of a subject, or a pro-drug, which becomes bioactive or more bioactive after it has been placed in a predetermined physiological environment.

The term "tacky polymer" refers to a polymer that has at least one surface that is tacky at room temperature. In one aspect, a "tacky polymer" can act like an adhesive. A "tacky polymer" can be designed to be capable of adhering to a material or article, such as an implant device, release liner, or tissue or fluid of a subject.

The term "elastic" refers to a resilient material that can be deformed (*e.g.,* stretched or expanded) and subsequently recover from the deformation, *i.e.,* substantially return to its initial state, prior to the deformation.

The term "band" refers to a material having at least one continuous elongate surface that forms a loop.

The term "adhering terpolymer" refers to a terpolymer that can adhere to a contacting surface. The "adhering terpolymer" can be any terpolymer capable of sticking to a surface for a desired time period. In one aspect, an "adhering terpolymer" can act like, or be, an adhesive, a gel, a wax or waxy polymer, a Vaseline® like material, a viscous terpolymer, or a tacky terpolymer.

Disclosed are compounds, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a number of different polymers and agents are disclosed and discussed, each and every combination and permutation of the polymer and agent are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this disclosure including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

The implantable composites and compositions described herein can be applied to an implant device, or to a tissue or fluid of a subject. The implantable composite or composition can release a bioactive agent into the subject. The composites and compositions described herein allow for controlled-release, extended-release, modified-release, sustained-release, pulsatile-release, delayed-release, or programmed-release of the bioactive agent. Implantable film-based composites, implantable elastic band composites, implantable flexible body composites, implantable suction cup composites, terpolymer compositions, and spray coating compositions are each disclosed and described herein below.

### i) Implantable Film-Based Composites

In one aspect, the implantable composite is an implantable film-based composite. In this aspect, the implantable composite comprises a biocompatible polymer film and a releasable bioactive agent; and a release liner affixed to the biocompatible polymer film. In a further aspect, the implantable composite comprises a biocompatible polymer film having at least a first surface comprising an adhesive on at least a portion thereof; wherein a release liner is affixed to the adhesive; and wherein a releasable bioactive agent is in or on the film. In a further aspect, an implantable composite comprises a biocompatible polymer film having at least a first surface, and a releasable bioactive agent; and an adhesive on at least a portion of the first surface; wherein the implantable composite has a length dimension and a width dimension, wherein the length dimension is substantially the same as the width dimension. In a further aspect, an implantable composite comprises a biocompatible polymer film having at least a first surface, and a releasable bioactive agent; an adhesive on at least a portion of the first surface; wherein the implantable composite has a shape that is not rectangular.

In one aspect, an implant device comprises at least a first implant device surface comprising a disclosed implantable film-based composite on at least a portion thereof, wherein the implantable film-based composite has a length dimension and a width dimension, wherein the length dimension is substantially the same as the width dimension. In a further aspect, an implant device comprises at least a first implant device surface comprising a disclosed implantable film-based composite on at least a portion thereof wherein the implantable film-based composite has a shape that is not rectangular.

Also disclosed are methods of applying an implantable film-based composite to an implant device, the method comprising securing an implantable film-based composite onto a surface of an implant device, substantially close to the time when the implant device is implanted in a subject.

In one aspect, the implantable film-based composite comprises a biocompatible polymer film having at least a first surface, and a releasable bioactive agent therein the composite; an adhesive on at least a portion of the first surface; and a release liner affixed to the adhesive.

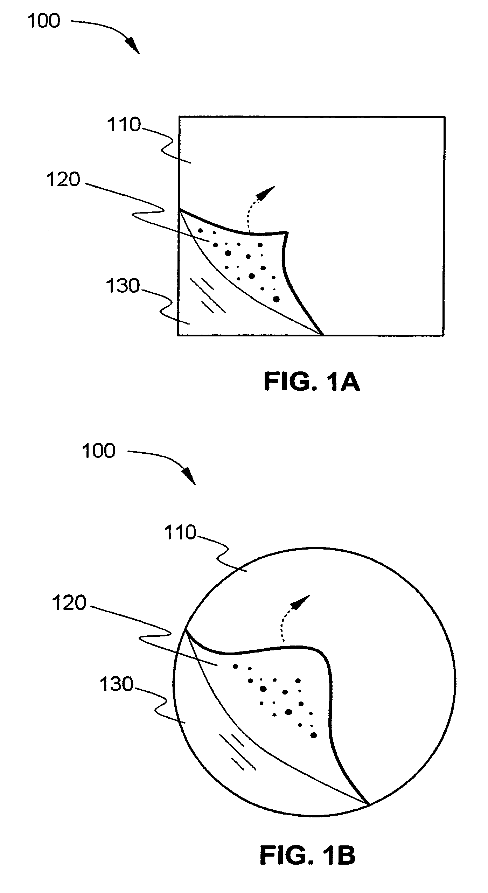

The implantable film-based composite can have any desired shape. For example, the implantable film-based composite can be substantially spherical, cylindrical, planar, or cubical. A planar implantable film-based composite can be substantially square, rectangular, circular, triangular, among other shapes. In one aspect, the implantable film-based composite can be square or rectangular. In a further aspect, the implantable film-based composite has a shape that is non-rectangular. As shown in Figure 1A, for example, an implantable film-based composite **100** can have a substantially squared shape, with the length of the composite approximately equal to the width of the composite. As shown in Figure 1A, the implantable film-based composite **100** comprises a polymer film **110** having at least a first surface **120.** The polymer film **110** comprises the releasable bioactive agent (not shown). An adhesive (not shown) can be optionally present on at least a portion of a first surface **120,** or on the entire first surface **120,** of the polymer film. The adhesive allows the polymer film to adhere to an implant device, or to an internal tissue or fluid of a subject. Prior to the implantable film-based composite being implanted in a subject, the adhesive can be at least partially, or fully, covered with a release liner. As shown in Figure 1A, a release liner **130** is affixed to the adhesive present on the first surface **120** of the polymer film. In other alternative embodiments, the release liner is not present.

The implantable film-based composites can have any desired size. In general, the size selection of the implantable film-based composite can be influenced by the desired loading of the bioactive agent. Generally, the more bioactive agent that is desired, the larger the implantable film-based composite will be. The size can also be selected so as to provide the desired release properties of the polymer film. In addition, when the implantable film-based composite is applied to an implant device, the size of the implant device can be of importance when selecting the size of the implantable film-based composite. For example, it can be desirable for portions of the implant device surface to remain exposed. In these instances, the size of the implant can be selected so as to not completely cover the implant device surface.

Implantable film-based composites can occupy any desired volume, for example, volumes of from about 0.1 cm³ to about 30 cm³, including, for example, 1, 2, 3, 4, 5, 6, 7, 10, 12, 14, 15, 18, 20, 22, 25, 28, and 29 cm³. Smaller implantable film-based composites, for example, can occupy a volume of from about 0.1 mm³ to about 100 mm³, including, for example, 0.5, 1, 3, 5, 7, 9, 10, 12, 15, 20, 25, 30, 35, 40, 45, 50, 60, 70, 80, 85, or 90 mm³. In other aspects, the implantable film-based composite can be shaped so as to have a length and a width. The length can be greater than, less than, or equal to, the width of the implantable film-based composite. Suitable lengths and widths include, for example, 0.1 cm, or smaller, to about 30 cm, or larger, including without limitation 1, 2, 3, 4, 5, 6, 7, 10, 12, 14, 15, 18, 20, 22, 25, 28, and 29 cm. For example, the implantable film-based composite can have an area of from about 0.1 cm x 0.1 cm to about 30 cm x 30 cm, including, for example, 0.1 cm x 0.5 cm, 1 cm x 1 cm, 1 cm x 1.5 cm, 2 cm x 2 cm, 2 cm x 0.5 cm, 5 cm x 5 cm, 5 cm x 2 cm, 10 cm x 10 cm, 10 cm x 5 cm, 15 cm x 10 cm, 15 cm x 15 cm, 20 cm x 20 cm, 20 cm x 15 cm, 25 cm x 25 cm, or 30 cm x 20 cm. Smaller implantable film-based composites can have a length or width from about 0.1 mm, or smaller, to about 1 mm, or larger, including, for example, 0.2, 0.3, 0.4, 0.5, 0.6, 0.7, 0.8, or 0.9 mm. Such implants can have an area of from about 0.1 mm x 0.1 mm, 0.2 mm x 0.1 mm, 0.3 mm x 0.3 mm, 0.3 mm x 0.2 mm, 0.5 mm x 0.5 mm, 0.5 mm x 0.2 mm, 0.8 mm x 0.8 mm, or 0.9 mm x 0.9 mm.

In one aspect, wherein if the implantable film-based composite has a length dimension substantially larger than a width dimension and a width dimension substantially larger than a thickness dimension, then the width dimension is less than about 2 mm. In a further aspect, if the implantable film-based composite has a length dimension substantially larger than a width dimension and a width dimension substantially larger than a thickness dimension, then the width dimension is less than about 2 mm. In a still further aspect, the implantable film-based composite can have a length dimension that is substantially the same as the width dimension. In a further aspect, the implantable film-based composite does not have a length dimension substantially larger than a width dimension and a width dimension substantially larger than a thickness dimension.

In still other aspects the implantable film-based composite can be a non square or non rectangular shape, and as such comprise one or more dimensions that is from about 0.1 cm to about 30 cm, including without limitation 1, 2, 3, 4, 5, 6, 7, 10, 12, 14, 15, 18, 20, 22, 25, 28, and 29 cm. A triangle, for example, can comprise one or more dimensions in the aforementioned size ranges. In further aspects, the implantable film-based composites can be substantially circular (e.g., as shown in Figure 1B), and comprise a diameter from about 0.1 cm to about 30 cm, including, for example, 1, 2, 3, 4, 5, 6, 7, 10, 12, 14, 15, 18, 20, ₂2; 25,28, and 29 cm.

The polymer film can have any desired thickness. In one aspect, the polymer film is a thin film having a thickness of from about 1 nm, or less, to about 1000 nm, including without limitation those films having thicknesses of about 5 nm, 20 nm, 50 nm, 150 nm, 200 nm, 300 nm, 500 nm, 800 nm, or 900 nm. In a further aspect, the film has a thickness greater than about 1000 nm, including without limitation those films having thicknesses of from about 1000 nm to about 50 cm, or greater. For example, the film can have a thickness of about 2000 nm, 0.1 cm, 0.5 cm, 1 cm, 5 cm, 20 cm, 30 cm, 40 cm, or 50 cm. It is to be understood that the film does not have to be, but can be, planar. Thus, in various aspects, the film may have varying heights at different regions of the film. As such, the film can comprise any shape, as discussed above, depending on the desired shape of the implantable film-based composite.

The polymer used with the implantable composites can be any suitable biocompatible and biodegradable or non-biodegradable polymer. The polymers disclosed herein can be homopolymers or copolymers. The polymers can be block or blocky co- or ter- polymers, random co- or ter- polymers, star polymers, or dendrimers. Any desired molecular weight polymer can be used, depending on the desired properties of the implantable film-based composite. In certain aspects, if a high strength implantable film-based composite is desired, then high molecular weight polymers can be used, for example, to meet strength requirements. In other aspects, low or medium molecular weight polymers can be used when, for example, when resorption time of the polymer, rather than material strength is desired.

The molecular weight of the polymer can be selected so as to provide a desired property of the implantable film-based composite. In certain aspects, the polymer film can be provided by forming a film of the polymer. In such aspects, the molecular weight should be high enough so that it forms satisfactory films. Usually, a molecular weight greater than 10,000 daltons can achieve this. However, since the properties of the film are also partially dependent on the particular polymeric material being used, it is difficult to specify an appropriate molecular weight range for all polymers. The molecular weight of a polymer is also important from the point of view that molecular weight influences the biodegradation rate of the polymer. For a diffusional mechanism of bioactive agent release, the polymer should remain intact until all of the drug is released from the polymer and then degrade. The drug can also be released from the polymer as the polymer bioerodes. By an appropriate selection of polymeric materials a polymer formulation can be made such that the resulting biocompatible polymer exhibits both diffusional release and biodegradation release properties. Molecular weights can be measured by methods known in the art, including gel permeation chromatography, viscosity, light-scattering, among other methods.

The biodegrable polymer film can be formulated so as to degrade within a desired time interval, once present in a subject. In some aspects, the time interval can be from about less than one day to about 1 month. Longer time intervals can extend to 6 months, including for example, polymer matrices that degrade from about ≥0 to about 6 months, or from about 1 to about 6 months. In other aspects, the polymer can degrade in longer time intervals, up to 2 years or longer, including, for example, from about ≥0 to about 2 years, or from about 1 month to about 2 years.

The desired bioactive agent release mechanism can influence the selection of the polymer. A biocompatible polymer can be selected so as to release or allow the release of a bioactive agent therefrom at a desired lapsed time after the implantable film-based composite has been implanted into a subject. For example, the polymer can be selected to release or allow the release of the bioactive agent prior to the bioactive agent beginning to diminish its activity, as the bioactive agent begins to diminish in activity, when the bioactive agent is partially diminished in activity, for example at least 25%, at least 50% or at least 75% diminished, when the bioactive agent is substantially diminished in activity, or when the bioactive agent is completely gone or no longer has activity.

In one aspect, the polymer can be one or more of polyesters, polyhydroxyalkanoates, polyhydroxybutyrates, polydioxanones, polyhydroxyvalerates, polyanhydrides, polyorthoesters, polyphosphazenes, polyphosphates, polyphosphoesters, polydioxanones, polyphosphoesters, polyphosphates, polyphosphonates, polyphosphates, polyhydroxyalkanoates, polycarbonates, polyalkylcarbonates, polyorthocarbonates, polyesteramides, polyamides, polyamines, polypeptides, polyurethanes, polyalkylene alkylates, polyalkylene oxalates, polyalkylene succinates, polyhydroxy fatty acids, polyacetals, polycyanoacrylates, polyketals, polyetheresters, polyethers, polyalkylene glycols, polyalkylene oxides, polyethylene glycols, polyethylene oxides, polypeptides, polysaccharides, or polyvinyl pyrrolidones. Other non-biodegradable but durable polymers include without limitation ethylene-vinyl acetate co-polymer, polytetrafluoroethylene, polypropylene, polyethylene, and the like. Likewise, other suitable non-biodegradable polymers include without limitation silicones and polyurethanes.

In a further aspect, the polymer can be a poly(lactide), a poly(glycolide), a poly(lactide-co-glycolide), a poly(caprolactone), a poly(orthoester), a poly(phosphazene), a poly(hydroxybutyrate) or a copolymer containing a poly(hydroxybutarate), a poly(lactide-co-caprolactone), a polycarbonate, a polyesteramide, a polyanhydride, a poly(dioxanone), a poly(alkylene alkylate), a copolymer of polyethylene glycol and a polyorthoester, a biodegradable polyurethane, a poly(amino acid), a polyamide, a polyesteramide, a polyetherester, a polyacetal, a polycyanoacrylate, a poly(oxyethylene)/poly(oxypropylene) copolymer, polyacetals, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations, or blends thereof.

In a still further aspect, useful biocompatible polymers are those that comprise one or more residues of lactic acid, glycolic acid, lactide, glycolide, caprolactone, hydroxybutyrate, hydroxyvalerates, dioxanones, polyethylene glycol (PEG), polyethylene oxide, or a combination thereof. In a still further aspect, useful biocompatible polymers are those that comprise one or more residues of lactide, glycolide, caprolactone, or a combination thereof.

In one aspect, useful biodegradable polymers are those that comprise one or more blocks of hydrophilic or water soluble polymers, including, but not limited to, polyethylene glycol, (PEG), or polyvinyl pyrrolidone (PVP), in combination with one or more blocks another biocompabible or biodegradable polymer that comprises lactide, glycolide, caprolactone, or a combination thereof.

In specific aspects, the biodegradable polymer can comprise one or more lactide residues. The polymer can comprise any lactide residue, including all racemic and stereospecific forms of lactide, including, but not limited to, L-lactide, D-lactide, and D,L-lactide, or a mixture thereof. Useful polymers comprising lactide include, but are not limited to poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide polymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL- polymers. In addition to copolymers comprising glycolide and DL-lactide or L-lactide, copolymers of L-lactide and DL-lactide are commercially available. Homopolymers of lactide or glycolide are also commercially available.

When the biodegradable polymer is poly(lactide-co-glycolide), poly(lactide), or poly(glycolide), the amount of lactide and glycolide in the polymer can vary. In a further aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In a further aspect, the biodegradable polymer can be poly(lactide), 95:5 poly(lactide-co-glycolide) 85:15 poly(lactide-co-glycolide), 75:25 poly(lactide-co-glycolide), 65:35 poly(lactide-co-glycolide), or 50:50 poly(lactide-co-glycolide), where the ratios are mole ratios.

In a further aspect, the polymer can be a poly(caprolactone) or a poly(lactide-co-caprolactone). In one aspect, the polymer can be a poly(lactide-caprolactone), which, in various aspects, can be 95:5 poly(lactide-co-caprolactone), 85:15 poly(lactide-co-caprolactone), 75:25 poly(lactide-co- caprolactone), 65:35 poly(lactide-co- caprolactone), or 50:50 poly(lactide-co- caprolactone), where the ratios are mole ratios.

In one aspect, the polymer film can comprise a terpolymer. In one aspect, the terpolymers are those terpolymers disclosed in (U.S.Patent Publication No. 2009/0124535).

It is understood that any combination of the aforementioned biodegradable polymers can be used, including, but not limited to, copolymers thereof, mixtures thereof, or blends thereof. Likewise, it is understood that when a residue of a biodegradable polymer is disclosed, any suitable polymer, copolymer, mixture, or blend, that comprises the disclosed residue, is also considered disclosed. To that end, when multiple residues are individually disclosed (*i.e.,* not in combination with another), it is understood that any combination of the individual residues can be used.

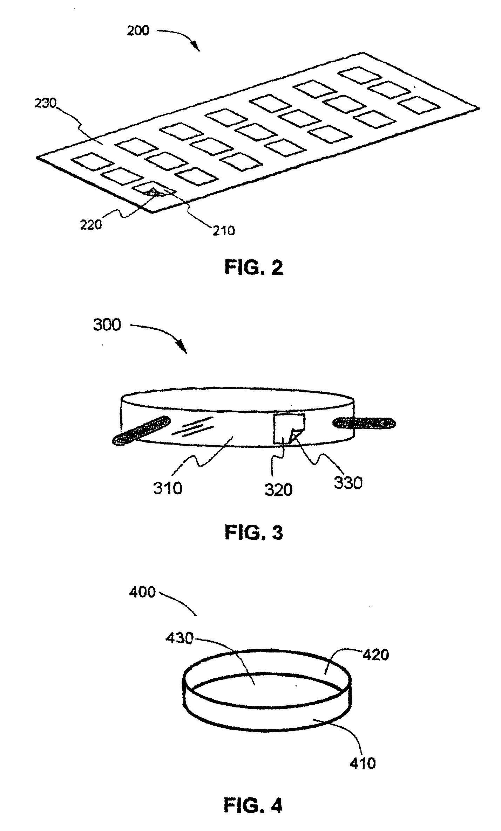

Also disclosed are articles and kits comprising the implantable film-based composites. An exemplary article **200** is shown in Figure 2, which shows a planar release liner **230** comprising a plurality of implantable film-based composites. As shown, each implantable film-based composite comprises a polymer film **210** having at least a first surface **220** that optionally comprises an adhesive (not shown). The adhesive can be affixed to a release liner **230.** In this example, the article comprises a plurality of implantable film-based composites that all share the release liner **230.** However, alternate embodiments include kits that are a package of a plurality of implantable film-based composites, such as those depicted in Figure 1A and Figure 1B, in a package. Also disclosed are kits comprising a mixture of the same or different implantable film-based composites. For example, the kit can comprise several sets of implantable film-based composites, each having a different size. Such a kit may be useful for point of use applications of the implantable film-based composites, wherein one kit, for example, can provide implantable film-based composites that are compatible in size with a number of different implant devices.

In one aspect, an adhesive can be present on a surface of a disclosed implantable film-based composite. In certain aspects, the polymer film itself can be a tacky polymer film, which functions as an adhesive to which a release liner, implant device, or tissue or fluid of subject can directly adhere. Methods of making the above disclosed polymers tacky are known in the art. Addititives for example, can be added to provide a tacky polymer that can be adhesive. In one aspect, tacky polymers can be those that comprise a *T*g of less than about room temperature, including those polymers disclosed above which have glass transition temperatures of less than about room temperature.

In various aspects of the implantable film-based composites, the polymer film does not have to be, but can be, comprised of a tacky polymer film. Thus, in a further aspect, an adhesive, separate from the polymer film, can be on a polymer film. The adhesive can be any desired adhesive. Suitable adhesives include without limitation thermoplastics, glycoproteins, mucopolysaccharides, bioadhesives, carbohydrates, starches, dextrin, sugars, gelatin, epoxy, acrylics, rubber, silicones, polyurethanes, pressure sensitive adhesives, polyesters, polyethers, polychloroprene, natural gums, peroxides, silanes, isocyanates, or combinations, mixtures, and blends thereof.

In one aspect, the adhesive can be a biocompatible or biodegradable adhesive, including without limitation, poly(lactide-co-caprolactone), poly(glycolide-co-caprolactone), or combinations, mixtures, and blends thereof.

The adhesive can be applied to the first surface of the polymer film through methods known in the art. Adhesives can be applied, for example, through spin-coating, drop-casting, brushing, or spraying an adhesive composition onto the first surface of the polymer film.

As discussed above, in one aspect, the implantable film-based composite comprises a release liner. The release liner can be any suitable release liner. The release liner is typically a temporary release liner that is removed from the polymer film of the implantable film-based composite prior to the implantable film-based composite being implanted into a subject. As such, it is preferred that the temporary release liner not leave behind any material in a quantity that could be harmful to a subject.

Suitable release liners are those that are made of materials that permit the release liner to be easily stripped or peeled away from the adjacent adhesive. Examplary release liners are those that are comprised of paper and/or a plastic material. Typically, such release liners are made from polymers such as polyesters or polyethylenes which are coated with materials such as silicone or fluorinated hydrocarbons that reduce the adhesiveness between the release liner and the adjacent adhesive. Other suitable release liners include paper, such as kraft paper, that is covered with a silicone material, which permits the easy release of the liner from the adhesive. Release liner materials are available commercially, for example, polyethylene is commercially available from 3M^{®}.

In one aspect, the release liner is the polymer film. That is, the first surface of the polymer film is adhered to an opposing second surface of the polymer film with an adhesive. For example, the implantable film-based composite can be configured as a roll of tape, wherein the second opposing surface of the polymer film functions as the release liner. In other aspects, the release liner is not the polymer film. That is, the first surface of the polymer film is not adhered to an opposing second surface of the polymer film. Thus, in some aspects, the implantable film-based composite is not a roll of tape, wherein the second opposing surface of the polymer film functions as the release liner.

In one aspect, the implant device comprises at least a first implant device surface comprising an implantable film-based composite on at least a portion thereof, wherein if the implantable film-based composite has a length dimension substantially larger than a width dimension and a width dimension substantially larger than a thickness dimension, then the width dimension is less than about 2 mm.

The implant device can comprise any shape, such as a rod, a fiber, a cylinder, a bead, a ribbon, a disc, a wafer, a free-formed shaped solid, or a variety of other shaped solids. The device can have any regular or irregular shape and can have any cross section like circular, rectangular, triangular, oval, and the like. In a further aspect, the device comprises a cylindrical shape, such as a typical shape of an implantable pump.

The implant can be comprised of any suitable material, such as a metal (e.g., titanium), metal composite, organic material, polymeric, or even ceramic material. The surface of the implant can be any shaped surface, and may have a porous, beaded or meshed ingrowth surface, as can be present in certain implants.

The implant device can be any type of medical implant. The implant devices can include, for example, implants for drug delivery, including drug delivery pumps; orthopedic implants, including spinal implants, implants for osseointegration or bone repair; medical stents, including stents with inherent drug delivery capability; prosthetic implants, including breast implants, muscle implants, and the like; dental implants; ear implants, including cochlear implants and hearing devices; cardiac implants including pacemakers, catheters, etc.; space filling implants; bioelectric implants; neural implants; internal organ implants, including dialysis grafts; defribrillators; monitoring devices; recording devices; stimulators, including deep brain stimulators, nerve stimulators, bladder stimulators, and diaphragm stimulators; implantable identification devices and information chips; artificial organs; drug administering devices; implantable sensors/biosensors; screws; tubes; rods; plates; or artificial joints.

In a further aspect, the implant device can be at least one of a pump, pacemaker, defribrillator, or stimulator, including deep brain stimulators, nerve stimulators, bladder stimulators, and diaphragm stimulators.

With reference to Figure 3 an implant device **300** comprises a first implant device surface **310** that comprises an implantable composite comprised of a polymer film **320,** which is adhered to the first implant device surface by a first surface **330** of the polymer film **320.** The device shown in the Figure 3 is an implantable pump. Once the implant device is present in a subject, the polymer film can degrade, allowing the bioactive agent to be released in or near the tissue that is adjacent the implant site. If desired, a plurality of implantable film-based composites can be applied to the implant device.

Other implant devices that may benefit when used with the disclosed implantable film-based composites include those with one or more active surfaces, *e.g.,* a surface that enhances a connection between a tissue or fluid and the implant device, or a surface that allows for or enhances wound healing. The disclosed implantable film-based composites can be effective when applied to only a portion of the implant device, allowing for any active surface to remain exposed and functional when the implant device is implanted in a subject.

As discussed above, it can be desirable to deliver a bioactive agent at or near the tissue adjacent an implant site. The bioactive agent can help prevent some of the problems associated with implants, such as infection, or enhance the function of the implant itself. It can also be desirable to avoid pre-manufacturing an implant device with bioactive agent releasing capability, as discussed above. It should be appreciated that the composites, methods, and kits disclosed herein can allow for a point of use application of an implantable film-based composite onto the surface of an implant device, thus obviating the need to pre-manufacture implant devices having bioactive agent releasing capability.

In one aspect, an implantable film-based composite can be applied to an implant device surface close to or during the time of use. For example, an implantable film-based composite can be applied to an implant device by securing the implantable film-based composite onto the surface of the implant device, substantially close to the time when the implant device is implanted in a subject. In one aspect, the implantable film-based composite can be applied to an implant device in an operating suite, for example, by a physician or nurse.

The implantable film-based composite can be secured to the surface of the implant device prior to or after the time when the implant device is implanted in the subject. In one aspect, the implant device comprising the implantable film-based composite can be implanted into the subject. In a further aspect, the implant device can be implanted into the subject, and then the implantable film-based composite can be applied to the surface of the implant device. When implanting smaller implants, it may be beneficial to first apply the implantable film-based composite to the implant device surface.

In one aspect, the implantable film-based composite can be secured to the surface of the implant device on the same day (*i.e.,* within 24 hours) of the implant surgery, including, for example, within 23 hours, 20 hours, 15 hours, 10 hours, 5 hours, 3 hours, 2 hours, 1 hour, 30 minutes, 15 minutes, 10 minutes, 5 minutes, 2 minutes, 30 seconds, or during the implant surgery itself.

If desired, the implantable film-based composite itself, with or without an implant device, can be implanted onto or in a tissue or fluid of a subject. In one aspect, the implantable film-based composite can be implanted onto or in a tissue or fluid that is near or adjacent to an implant site, *i.e.,* a site where an implant device has been implanted, or near or adjacent to a desired implant site.

In one aspect, the disclosed methods can be used with implantable film-based composites comprising a releasable bioactive agent. In a further aspect, the methods can be used with implantable film-based composites comprising a polymer film having at least a first surface, and a releasable bioactive agent; and an adhesive on at least a portion, or all, of the first surface. In a still further aspect, the methods can be used with implantable film-based composites comprising a polymer film having at least a first surface, and a releasable bioactive agent, with an adhesive on at least a portion, or all, of the first surface, and a release liner affixed to the adhesive; wherein the release liner is removed from the adhesive before the implantable film-based composite is secured onto the surface of the implant device. The release liner can be removed by pealing the release liner away from the polymer film and/or adhesive, or by pealing the polymer film away from the release liner. The implantable film-based composite can then be applied to the surface of the implant, or to the tissue or fluid of the subject, by contacting the surface of the implant with the contacting side of the implantable film-based composite and optionally applying pressure to promote good adhesion. If a pressure sensitive adhesive is used, it can be desirable to apply pressure to the implantable film-based composite to promote good adhesion of the composite onto the implant device surface.

Also disclosed are implant devices comprising the implantable film-based composites. The term "device" is any formulation or article that is greater than 1 mm in length in at least one dimension of the device. The device can comprise a disclosed implantable composite. In a further aspect, the device has one dimension that is from 1 mm to 50 mm, 1.2 mm to 45 mm, 1.4 mm to 42 mm, 1.6 mm to 40 mm, 1.8 mm to 38 mm, or 2.0 mm to 36 mm, 5.0 mm to 33 mm, or 10 mm to 30 mm. In a further aspect, the device has one dimension that is greater than 3 cm, even up to or greater than 10 cm, 20 cm, or even 30 cm.

The implant device can be implanted in any desired subject. The subject can be a vertebrate, such as a mammal, a fish, a bird, a reptile, or an amphibian. The subject of the herein disclosed methods can be, for example, a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered.

Typically, before applying the implantable film-based composite to the implant device, the implant device surface can be cleaned or treated to remove any surface contaminants and to promote good adhesion of the polymer film. For example, the implantable composite and/or the implant device can be sterilized e.g. by autoclaving under water steam. The implantable film-based composite or implant device comprising the implantable film-based composite can then be implanted into the subject using known surgical techniques. In certain aspects, it can be desirable to store the implantable composites or kits comprising the composites in a sterilized container or package. In one aspect, the kit can comprise a sterilized package of the implantable composites.

### ii) Implantable Elastic Band Composites

In another aspect, the implantable composite is an elastic band composite comprising an elastic band having at least one continuous elongate surface and comprising a biocompatible polymer and a bioactive agent. In a further aspect, an implant device comprises a disclosed implantable elastic band composite contacting at least a portion of the implant device. Also disclosed are methods of applying an implantable elastic band composite to an implant device, the method comprising securing an implantable elastic band composite onto a surface of an implant device, substantially close to the time when the implant device is implanted in a subject.

In one aspect, the implantable elastic band composite comprises an elastic band having at least one continuous elongate surface and comprising a biocompatible polymer and bioactive agent disposed therein the matrix. In one aspect, the bioactive agent is disposed in the band and/or the polymer. In one aspect, the elastic band can be biocompatible or biodegradable. The implantable elastic band composites disclosed herein can act, and have properties like, a rubber band. As such, the implantable elastic band composite is typically a closed loop that can be attached around an implant device, or tissue or fluid of a subject.

With reference to Figure 4, an implantable elastic band composite **400** can comprise an elastic band **410** having at least one continuous elongate surface **420** that defines at least one opening **430** therethrough. The band **410** comprises the releasable bioactive agent (not shown) disposed therein the band or on a surface of the band. The elastic band can be expanded and applied to (e.g., expanded and placed around) an implant device, or to an internal tissue or fluid of a subject. In one aspect, the at least one continuous elongate surface can have an opposed second continuous elongate surface. In a further aspect, the elastic band can be a single-layered elastic band.

The implantable elastic band composites can have any desired size. In general, the size selection of the implantable elastic band composite can be influenced by the desired loading of the bioactive agent. Generally, the more bioactive agent that is desired, the larger the implantable elastic band composite will be. The size can also be selected so as to provide the desired release properties of the polymer. In addition, when the implantable elastic band composite is applied to an implant device, the size of the implant device can be of importance when selecting the size of the implantable elastic band composite. For example, it can be desirable for portions of the implant device surface to remain exposed. In these instances, the size of the implant can be selected so as to not completely cover the implant device surface.

In one aspect, the opening of the implantable elastic band composites can have any desired size, when not expanded, including without limitation diameters of from about 1 cm to about 50 cm or greater, from about 5 cm to about 25 cm, or from about 7 cm to about 15 cm, including those implantable elastic band composites comprising one or more openings having a diameter of about 2, 3, 4, 5, 6, 7, 8, 9, 10, 11, 12, 13, 14, 15, 16, 17, 18, 19, 20, 30, 40, or 45 cm, or larger. Additionally, the implantable elastic band composites can comprise one or more openings having a diameter less than about 1 cm, including for example, from about 0.1 cm to about 1 cm. In a further aspect, the implantable elastic band composites can be stretched to any desired size, depending on the degree of elasticity of the elastic band, discussed below.

In one aspect, the elastic band can comprise an elastic polymer. Elastic polymers are those that can undergo a reversible elongation at a relatively low stress or strain. Such polymers include without limitation amorphous polymers, polymers with low glass transition temperatures (*T*_{g}), polymers with high polymer chain mobility, cross-linked polymers, or a combination thereof. In one aspect, suitable polymers are those that are substantially amorphous and have a low *T*_{g}. Suitable glass transition temperatures include, without limitation, about 25 °C or less, about 15 °C or less, about 10 °C or less, about 0 °C or less, about -10 °C or less, about -20 °C or less, about -30 °C or less, about -40 °C or less, about -50 °C or less, about -60 °C or less, about -70 °C or less, or less than -70 °C. In a further aspect, suitable polymers are those that are cross-linked. The degree of cross-linking for an elastic polymer can be typically selected based on the desired elasticity of the polymer. Generally, at least some degree of cross-linking can be useful to achieve relatively rapid deformation and nearly complete reversibility.

Suitable elastic polymers can have a relatively low initial force modulus (e.g., less than 100 N/cm), so that the polymer can be expanded without an inconvenient amount of force. Typically, however, the force modulus should increase beyond the initial force modulus upon expansion of the polymer. In certain aspects, to maintain high strength at a high expansive force, a polymer can be cross-linked, as discussed above, and/or can comprise biocompatible filler materials which add strength to the matrix. Additionally, the polymer can be engineered to undergo a small amount of crystallization at high elongation, which can add strength to the polymer during forceful expansion. Other additives can be added to the polymer to aid in the elastic effect of the band, such as, for example, mineral oils, polyethylene glycol (PEG), fatty acids, carbon black, silica, kaolin, calcium carbonate, plastizers, solvents, or a combination or mixture thereof.

The polymer used with the implantable elastic band composites can comprise any biocompatible and biodegradable or non-biodegradable polymer. The polymers disclosed herein can be homopolymers or copolymers. The polymers can be block or blocky co- or ter- polymers, random co- or ter- polymers, star polymers, or dendrimers. Any desired molecular weight polymer can be used, depending on the desired properties of the implantable elastic band composite. In certain aspects, if a high strength elastic band composite is desired, then high molecular weight polymers can be used, for example, to meet strength requirements. In other aspects, low or medium molecular weight polymers can be used when, for example, when resorption time of the polymer, rather than material strength is desired. In one aspect, the polymer forms the elastic band.

The molecular weight of the polymer can be selected so as to provide a desired property of the elastic band composite. In certain aspects, the polymer can be provided by forming a molded composite of the polymer. In such aspects, the molecular weight should be such to allow a sufficient molded composite to form. The molecular weight should also be suitable to allow the polymer to be resiliently expanded. The molecular weight of a polymer is also important from the point of view that molecular weight influences the biodegradation rate of the polymer. For a diffusional mechanism of bioactive agent release, the polymer should remain intact until all of the drug is released from the polymer and then degrade. The drug can also be released from the polymer as the polymer bioerodes. By an appropriate selection of polymeric materials a polymer formulation can be made such that the resulting biodegradable polymer exhibits both diffusional release and biodegradation release properties. Molecular weights can be measured by methods known in the art, including gel permeation chromatography, viscosity, light-scattering, among other methods.

The biodegrable polymer can be formulated so as to degrade within a desired time interval, once present in a subject. In some aspects, the time interval can be from about less than one day to about 1 month. Longer time intervals can extend to 6 months, including for example, polymer matrices that degrade from about ≥0 to about 6 months, or from about 1 to about 6 months. In other aspects, the polymer can degrade in longer time intervals, up to 2 years or longer, including, for example, from about ≥0 to about 2 years, or from about 1 month to about 2 years.

The desired bioactive agent release mechanism can influence the selection of the polymer for the elastic band. A biodegradable polymer can be selected so as to release or allow the release of a bioactive agent therefrom at a desired lapsed time after the elastic band composite has been implanted into a subject. For example, the polymer can be selected to release or allow the release of the bioactive agent prior to the bioactive agent beginning to diminish its activity, as the bioactive agent begins to diminish in activity, when the bioactive agent is partially diminished in activity, for example at least 25%, at least 50% or at least 75% diminished, when the bioactive agent is substantially diminished in activity, or when the bioactive agent is completely gone or no longer has activity.

In one aspect of the elastic band composite, the polymer can be one or more of polyesters, polyhydroxyalkanoates, polyhydroxybutyrates, polydioxanones, polyhydroxyvalerates, polyanhydrides, polyorthoesters, polyphosphazenes, polyphosphates, polyphosphoesters, polydioxanones, polyphosphoesters, polyphosphates, polyphosphonates, polyphosphates, polyhydroxyalkanoates, polycarbonates, polyalkylcarbonates, polyorthocarbonates, polyesteramides, polyamides, polyamines, polypeptides, polyurethanes, polyalkylene alkylates, polyalkylene oxalates, polyalkylene succinates, polyhydroxy fatty acids, polyacetals, polycyanoacrylates, polyketals, polyetheresters, polyethers, polyalkylene glycols, polyalkylene oxides, polyethylene glycols, polyethylene oxides, polypeptides, polysaccharides, or polyvinyl pyrrolidones. Other non-biodegradable but durable polymers include without limitation ethylene-vinyl acetate co-polymer, polytetrafluoroethylene, polypropylene, polyethylene, and the like. Likewise, other suitable non-biodegradable polymers include without limitation silicones and polyurethanes.

In a further aspect of the elastic band composite, the polymer can be a poly(lactide), a poly(glycolide), a poly(lactide-co-glycolide), a poly(caprolactone), a poly(orthoester), a poly(phosphazene), a poly(hydroxybutyrate) or a copolymer containing a poly(hydroxybutarate), a poly(lactide-co-caprolactone), a polycarbonate, a polyesteramide, a polyanhydride, a poly(dioxanone), a poly(alkylene alkylate), a copolymer of polyethylene glycol and a polyorthoester, a biodegradable polyurethane, a poly(amino acid), a polyamide, a polyesteramide, a polyetherester, a polyacetal, a polycyanoacrylate, a poly(oxyethylene)/poly(oxypropylene) copolymer, polyacetals, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations, or blends thereof. Other non-biodegradable but durable polymers include without limitation ethylene-vinyl acetate co-polymer, polytetrafluoroethylene, polypropylene, polyethylene, and the like. Likewise, other suitable non-biodegradable polymers include without limitation silicones and polyurethanes.

In a still further aspect of the elastic band composite, useful biodegradable polymers are those that comprise one or more residues of lactic acid, glycolic acid, lactide, glycolide, caprolactone, hydroxybutyrate, hydroxyvalerates, dioxanones, polyethylene glycol (PEG), polyethylene oxide, or a combination thereof. In a still further aspect, useful biodegradable polymers are those that comprise one or more residues of lactide, glycolide, caprolactone, or a combination thereof.

In one aspect, useful biodegradable polymers are those that comprise one or more blocks of hydrophilic or water soluble polymers, including, but not limited to, polyethylene glycol, (PEG), or polyvinyl pyrrolidone (PVP), in combination with one or more blocks another biocompabible or biodegradable polymer that comprises lactide, glycolide, caprolactone, or a combination thereof.

In specific aspects of the elastic band composites, the biodegradable polymer can comprise one or more lactide residues. The polymer can comprise any lactide residue, including all racemic and stereospecific forms of lactide, including, but not limited to, L-lactide, D-lactide, and D,L-lactide, or a mixture thereof. Useful polymers comprising lactide include, but are not limited to poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide polymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL- polymers. In addition to copolymers comprising glycolide and DL-lactide or L-lactide, copolymers of L-lactide and DL-lactide are commercially available. Homopolymers of lactide or glycolide are also commercially available.

When the biodegradable polymer is poly(lactide-co-glycolide), poly(lactide), or poly(glycolide), the amount of lactide and glycolide in the polymer can vary. In a further aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In a further aspect, the biodegradable polymer can be poly(lactide), 95:5 poly(lactide-co-glycolide) 85:15 poly(lactide-co-glycolide), 75:25 poly(lactide-co-glycolide), 65:35 poly(lactide-co-glycolide), or 50:50 poly(lactide-co-glycolide), where the ratios are mole ratios.

In a further aspect, the polymer can be a poly(caprolactone) or a poly(lactide-co-caprolactone). In one aspect, the polymer can a poly(lactide-caprolactone), which, in various aspects, can be 95:5 poly(lactide-co-caprolactone) 85:15 poly(lactide-co-caprolactone), 75:25 poly(lactide-co- caprolactone), 65:35 poly(lactide-co- caprolactone), or 50:50 poly(lactide-co- caprolactone), where the ratios are mole ratios.

In one aspect, the elastic band composite can comprise a terpolymer. In one aspect, the terpolymers are those terpolymers disclosed in U.S. Patent Application Serial No. 12/269135, filed November 12, 2008, (U.S. Patent Publication No. 2009/0124535) which is incorporated herein by this reference for all of its teachings of terpolymers and is considered part of this disclosure.

It is understood that any combination of the aforementioned biodegradable polymers can be used, including, but not limited to, copolymers thereof, mixtures thereof, or blends thereof. Likewise, it is understood that when a residue of a biodegradable polymer is disclosed, any suitable polymer, copolymer, mixture, or blend, that comprises the disclosed residue, is also considered disclosed. To that end, when multiple residues are individually disclosed (*i.e.,* not in combination with another), it is understood that any combination of the individual residues can be used. Further, any of the above polymers can be processed (*e.g*., cross-linked to a desired level, to achieve a resiliently expandable polymer. An additional cross-linking agent can be used, and/or radical, cation, or anion cross-linking of the existing polymer can be used.

In one aspect, an adhesive can be present on an outer surface of the disclosed elastic band composite, that will contact the implant device surface, or the tissue or fluid of the subject, such as surface **420** shown in Figure 4. In certain aspects, the polymer itself can be a tacky polymer, which functions as an adhesive to which an implant device, or tissue or fluid of subject can adhere. Methods of making the above disclosed polymers tacky are known in the art. Addititives for example, can be added to provide a tacky polymer that can be adhesive. In one aspect, tacky polymers can be those that comprise a *T*_{g} of less than about room temperature, including those polymers disclosed above which have glass transition temperatures of less than about room temperature. Thus, in certain aspects, the resiliently expandable polymer can contact a disclosed implant device, tissue or fluid of a subject both elastically and adhesively, through the use of a tacky polymer.

In a further aspect, an adhesive, separate from the polymer, can be on the resiliently expandable polymer. The adhesive can be any desired adhesive. Suitable adhesives include without limitation thermoplastics, glycoproteins, mucopolysaccharides, bioadhesives, carbohydrates, starches, dextrin, sugars, gelatin, epoxy, acrylics, rubber, silicones, polyurethanes, pressure sensitive adhesives, polyesters, polyethers, polychloroprene, natural gums, peroxides, silanes, isocyanates, or combinations, mixtures, and blends thereof.

In one aspect, the adhesive can be a biodegradable adhesive, including without limitation, poly(lactide-co-caprolactone), poly(glycolide-co-caprolactone), or combinations, mixtures, and blends thereof.

The adhesive can be applied to the first surface of the polymer through methods known in the art. Adhesives can be applied, for example, through spin-coating, drop-casting, brushing, or spraying an adhesive composition onto the first surface of the polymer.

Also disclosed are kits comprising the implantable elastic band composites. The kit can be comprised one or more disclosed implantable composites, in a package. In one aspect, the kit can comprise the same implantable composite. In another aspect, the kit can comprise a mixture of different implantable composites. For example, the kit can comprise several sets of implantable composites, each having a different size. Such a kit may be useful for point of use applications of the implantable composites, wherein one kit, for example, can provide implantable composites that are compatible in size with a number of different implant devices.

Also disclosed are implant devices comprising the implantable elastic band composites. The device can be any formulation or article that is greater than 1 mm in length in at least one dimension of the device. The device can comprise a disclosed implantable composite. In a further aspect, the device has one dimension that is from 1 mm to 50 mm, 1.2 mm to 45 mm, 1.4 mm to 42 mm, 1.6 mm to 40 mm, 1.8 mm to 38 mm, or 2.0 mm to 36 mm, 5.0 mm to 33 mm, or 10 mm to 30 mm. In a further aspect, the device has one dimension that is greater than 3 cm, even up to or greater than 10 cm, 20 cm, or even 30 cm.

In one aspect, the implant device comprises a disclosed implantable composite elastically contacting at least a portion of the implant device.

The implant device can comprise any shape, such as a rod, a fiber, a cylinder, a bead, a ribbon, a disc, a wafer, a free-formed shaped solid, or a variety of other shaped solids. The device can have any regular or irregular shape and can have any cross section like circular, rectangular, triangular, oval, and the like. In a further aspect, the device comprises a cylindrical shape, such as a typical shape of an implantable pump.

The implant can be comprised of any suitable material, such as a metal (e.g., titanium), metal composite, organic material, polymeric, or even ceramic material. The surface of the implant can be any shaped surface, and may have a porous, beaded or meshed ingrowth surface, as can be present in certain implants.

The implant device can be any type of medical implant. The implant devices can include, for example, implants for drug delivery, including drug delivery pumps; orthopedic implants, including spinal implants, implants for osseointegration or bone repair; medical stents, including stents with inherent drug delivery capability; prosthetic implants, including breast implants, muscle implants, and the like; dental implants; ear implants, including cochlear implants and hearing devices; cardiac implants including pacemakers, catheters, etc.; space filling implants; bioelectric implants; neural implants; internal organ implants, including dialysis grafts; defribrillators; monitoring devices; recording devices; stimulators, including deep brain stimulators, nerve stimulators, bladder stimulators, and diaphragm stimulators; implantable identification devices and information chips; artificial organs; drug administering devices; implantable sensors/biosensors; screws; tubes; rods; plates; or artificial joints.

In a further aspect, the implant device can be at least one of a pump, pacemaker, defribrillator, or stimulator, including a deep brain stimulator, nerve stimulator, bladder stimulator, or diaphragm stimulator.

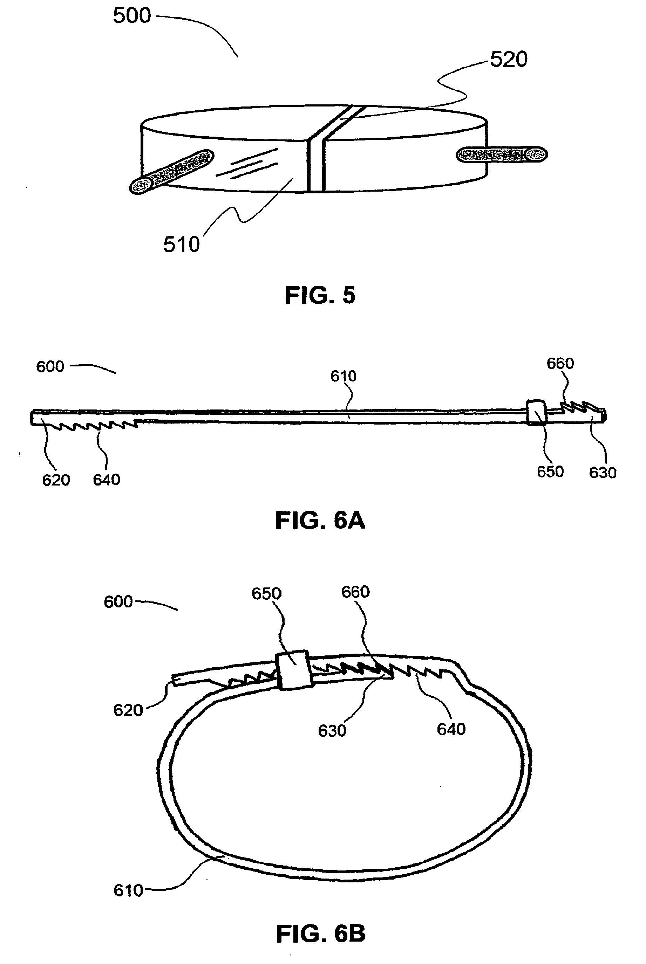

With reference to Figure 5, an implant device **500** can comprise a first implant device surface **510** that comprises an implantable elastic band composite comprised of a elastic band **520,** which is contacting at least a portion, or all, of the device **500.** The device shown in the Figure 5 is an implantable pump.

Once the implant device is present in a subject, the polymer can degrade, allowing the bioactive agent to be released in or near the tissue that is adjacent the implant site. If desired, a plurality of implantable composites can be applied to the implant device. Additionally, once the implantable composite has served its intended purpose, the implantable composite may fall off the implant device, into adjacent tissues or fluids.

Other implant devices that may benefit when used with the disclosed implantable composites include those with one or more active surfaces, *e.g.,* a surface that enhances a connection between a tissue or fluid and the implant device, or a surface that allows for or enhances wound healing. The disclosed implantable composites can be effective when applied to only a portion of the implant device, allowing for any active surface to remain exposed and functional when the implant device is implanted in a subject.

As discussed above, it can be desirable to deliver a bioactive agent at or near the tissue adjacent an implant site. The bioactive agent can help prevent some of the problems associated with implants, such as infection, or enhance the function of the implant itself. It can also be desirable to avoid pre-manufacturing an implant device with bioactive agent releasing capability, as discussed above. It should be appreciated that the composites, methods, and kits disclosed herein can allow for a point of use application of an implantable composite onto the surface of an implant device, thus obviating the need to pre-manufacture implant devices having bioactive agent releasing capability.

In one aspect, an implantable elastic band composite can be applied to an implant device surface close to or during the time of use. For example, an implantable composite can be applied to an implant device by securing the implantable composite onto the surface of the implant device, substantially close to the time when the implant device is implanted in a subject. In one aspect, the implantable composite can be applied to an implant device in an operating suite, for example, by a physician or nurse.

The implantable elastic band composite can be secured to the surface of the implant device prior to, at, or after the time when the implant device is implanted in the subject. In one aspect, the implant device comprising the elastic band composite can be implanted into the subject. In a further aspect, the implant device can be implanted into the subject, and then the elastic band composite can be applied to the surface of the implant device. When implanting smaller implants, it may be beneficial to first apply the elastic band composite to the implant device surface.

In one aspect, the elastic band composite can be secured to the surface of the implant device on the same day (*i.e.,* within 24 hours) of the implant surgery, including, for example, within 23 hours, 20 hours, 15 hours, 10 hours, 5 hours, 3 hours, 2 hours, 1 hour, 30 minutes, 15 minutes, 10 minutes, 5 minutes, 2 minutes, 30 seconds, or during the implant surgery itself.

If desired, the elastic band composite itself, with or without an implant device, can be implanted onto or in a tissue or fluid of a subject. In one aspect, the elastic band composite can be implanted onto or in a tissue or fluid that is near or adjacent to an implant site, *i.e.,* a site where an implant device has been implanted, or near or adjacent to a desired implant site.

Typically, before applying the elastic band composite to the implant device, the implant device surface can be cleaned or treated to remove any surface contaminants and to promote good adhesion of the polymer. For example, the elastic band composite and/or the implant device can be sterilized *e.g.* by autoclaving under water steam. However, in some aspects, care may be needed to avoid irreversibility deforming the resiliently expandable polymer. The elastic band composite or implant device comprising the elastic band composite can then be implanted into the subject using known surgical techniques. In certain aspects, it can be desirable to store the elastic band composites or kits comprising the composites in a sterilized container or package. In one aspect, the kit can comprise a sterilized package of the elastic band composites.

The disclosed methods can be used with any of the disclosed elastic band composites comprising a releasable bioactive agent. In one aspect, the method comprises expanding the resiliently expandable polymer, thereby increasing the diameter of the opening defined by the at least one polymer surface. The degree of expansion will typically be influenced by the size of the implantable device or tissue that will receive the elastic band composite. Once the polymer is expanded, the elastic band composite can be placed around an implant, tissue, or fluid, followed by a reduction of the expansive force, to allow the elastic band composite to relax and elastically contact at least a portion or the entire surface of the implant device. Optionally, an adhesive can be applied to a portion or the entire first surface of the polymer, or to a portion or the entire implant device surface, to aid in securing the elastic band composite to the implant device.

The implant device can be implanted in any desired subject. The subject can be a vertebrate, such as a mammal, a fish, a bird, a reptile, or an amphibian. The subject of the herein disclosed methods can be, for example, a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered.

### iii) Implantable Flexible Body Composites

In one aspect, the implantable composite is an implantable flexible body composite. The flexible body composites can be applied to an implant device, or to a tissue or fluid of a subject. The flexible body composites can release a bioactive agent into the subject. The composites described herein allow for controlled-release, extended-release, modified-release, sustained-release, pulsatile-release, delayed-release, or programmed-release of the bioactive agent.

In one aspect, the flexible body composite comprises a substantially flexible elongate body having a first end and a second end and comprising a biocompatible polymer having a releasable bioactive agent; wherein a first portion of the substantially flexible elongate body can be connected to a second portion of the substantially flexible elongate body to form a substantially continuous loop.

In a further aspect, a flexible body composite comprises a substantially flexible elongate body having a first end and a second end; and a means for connecting a first portion of the substantially flexible elongate body to a second portion of the substantially flexible elongate body, thereby forming a substantially continuous loop; wherein the substantially flexible elongate body comprises a biocompatible polymer and a releasable bioactive agent.

The flexible body composites can have any desired size. In general, the size selection of the flexible body composite can be influenced by the desired loading of the bioactive agent. Generally, the more bioactive agent that is desired, the larger the flexible body composite will be. The size can also be selected so as to provide the desired release properties of the substantially flexible elongate body. In addition, when the flexible body composite is applied to an implant device, the size of the implant device can be of importance when selecting the size of the flexible body composite. For example, it can be desirable for portions of the implant device surface to remain exposed. In these instances, the size of the implant can be selected so as to not completely cover the implant device surface.

The flexible body composites can have any desired size. For example, the flexible body composites, wherein the first end is connected to the second portion, can have diameters including without limitation of from about 1 cm to about 50 cm or greater, from about 5 cm to about 25 cm, or from about 7 cm to about 15 cm, including those flexible body composites comprising one or more openings having a diameter of about 2, 3, 4, 5, 6, 7, 8, 9, 10, 11, 12, 13, 14, 15, 16, 17, 18, 19, 20, 30, 40, or 45 cm, or larger. Additionally, the flexible body composites can have diameters of less than about 1 cm, including for example, from about 0.1 cm to about 1 cm. Likewise, the flexible body composites, wherein the first portion is not connected to the second portion, can have any desired length. In one aspect, the flexible body composites can have a length (*i.e.,* the distance from the first end to second end) of from about 1 cm to about 500 cm or greater, from about 5 cm to about 400 cm, from about 20 cm to about 200 cm, or from about 50 cm to about 100 cm, including those flexible body composites having lengths of about 2, 3, 4, 5, 8, 10, 20, 30, 40, 50, 60, 70, 80, 90, 100, 150, 200, 250, 300, 350, 400, 450, or 500 cm or greater.

In one aspect, a first portion of the substantially flexible elongate body can be connected to a second portion of the substantially flexible elongate body to form a substantially continuous loop. In a further aspect, the substantially flexible elongate body comprises a means for connecting the first portion of the body to the second portion of the body. The mechanical connection, or the mechanical connecting means, can comprise any desired connection. In one aspect, the mechanical connection, or the mechanical connecting means can comprise a zip tie connection, a cable tie connection, a locking connection, including for example wherein the first portion is twisted around the second portion, similarly to a twist tie connection, a snapping connection, a zipper or zipper-like connection, or any other connection known in the art.

In one aspect, the mechanical connection, or mechanical connecting means, comprises a zip tie connection. With reference to Figure 6A and 6B, the flexible body composite **600** can comprise a first portion **620** that comprises a series of ridges **640** along at least a portion, or all, of the first portion **620,** and wherein the second portion **630** comprises a receiving opening **650** comprising a ridged engaging tooth, or teeth, **660** for securely engaging at least a portion, or all, of the series of ridges **640** when the first portion **620** is inserted into the receiving opening **650** of the second portion **630.** As shown in Figure 6B, the first portion **620** is inserted into the receiving opening **650** of the second portion **630,** and engaged by one or more ridged engaging teeth **660** of the second portion.

In one aspect, the substantially flexible elongate body can comprise a semi-rigid polymer, or a polymer that is not an elastomer, but also not as rigid as a hard fiber. In some aspects, the substantially flexible elongate body should be flexible enough to allow for the flexible body composite to be mechanically secured to an implant device, tissue or fluid of a subject. In one aspect, the substantially flexible elongate body can be a plastic, such as a flexible plastic. Suitable flexible plastics are those that exhibit moderate to high ranges of crystallinity. Typically, a flexible plastic has a force modulus of from about 10,000 N/cm² to about 400,000 N/cm², with tensile strengths of from about 1000 N/cm² to about 10,000 N/cm². In one aspect, a flexible plastic can have an ultimate elongation percentage of from about 10% to about 1000%. For example, polyethylene is a typical flexible plastic with a tensile strength of about 2500 N/cm², a force modulus of about 20,000 N/cm², and an ultimate elongation of about 500 %. Other typical flexible plastics include without limitation polypropene and poly(hexamethylene adipamide). Additionally, those polymers discussed below can be engineered (*e.g.,* cross-linked, or processed with additives) to provide the desired mechanical properties of the flexible body composite, for example, flexibility and rigidity.

The substantially flexible elongate body used with the flexible body composites can comprise any biocompatible, biodegradable, or non-biodegradable polymer. The polymers disclosed herein can be homopolymers or copolymers. The polymers can be block or blocky co- or ter- polymers, random co- or ter- polymers, star polymers, or dendrimers. Any desired molecular weight polymer can be used, depending on the desired properties of the flexible body composite. In certain aspects, if a high strength flexible body composite is desired, then high molecular weight polymers can be used, for example, to meet strength requirements. In other aspects, low or medium molecular weight polymers can be used when, for example, when resorption time of the polymer, rather than material strength is desired.

The molecular weight of the polymer can be selected so as to provide a desired property of the flexible body composite. In certain aspects, the substantially flexible elongate body can be provided by forming a molded composite of the polymer. In such aspects, the molecular weight should be such to allow a sufficient molded composite to form. The molecular weight should also be suitable to allow the substantially flexible elongate body to be resiliently expanded. The molecular weight of a polymer is also important from the point of view that molecular weight influences the biodegradation rate of the polymer. For a diffusional mechanism of bioactive agent release, the polymer should remain intact until all of the drug is released from the polymer and then degrade. The drug can also be released from the polymer as the polymer bioerodes. By an appropriate selection of polymeric materials a polymer formulation can be made such that the resulting biodegradable polymer exhibits both diffusional release and biodegradation release properties. Molecular weights can be measured by methods known in the art, including gel permeation chromatography, viscosity, light-scattering, among other methods.

The substantially flexible elongate body can be formulated so as to degrade within a desired time interval, once present in a subject. In some aspects, the time interval can be from about less than one day to about 1 month. Longer time intervals can extend to 6 months, including for example, polymer matrices that degrade from about ≥0 to about 6 months, or from about 1 to about 6 months. In other aspects, the polymer can degrade in longer time intervals, up to 2 years or longer, including, for example, from about ≥0 to about 2 years, or from about 1 month to about 2 years.

The desired bioactive agent release mechanism can influence the selection of the polymer. A biodegradable polymer can be selected so as to release or allow the release of a bioactive agent therefrom at a desired lapsed time after the flexible body composite has been implanted into a subject. For example, the polymer can be selected to release or allow the release of the bioactive agent prior to the bioactive agent beginning to diminish its activity, as the bioactive agent begins to diminish in activity, when the bioactive agent is partially diminished in activity, for example at least 25%, at least 50% or at least 75% diminished, when the bioactive agent is substantially diminished in activity, or when the bioactive agent is completely gone or no longer has activity.

In one aspect, the polymer can be one or more of polyesters, polyhydroxyalkanoates, polyhydroxybutyrates, polydioxanones, polyhydroxyvalerates, polyanhydrides, polyorthoesters, polyphosphazenes, polyphosphates, polyphosphoesters, polydioxanones, polyphosphoesters, polyphosphates, polyphosphonates, polyphosphates, polyhydroxyalkanoates, polycarbonates, polyalkylcarbonates, polyorthocarbonates, polyesteramides, polyamides, polyamines, polypeptides, polyurethanes, polyalkylene alkylates, polyalkylene oxalates, polyalkylene succinates, polyhydroxy fatty acids, polyacetals, polycyanoacrylates, polyketals, polyetheresters, polyethers, polyalkylene glycols, polyalkylene oxides, polyethylene glycols, polyethylene oxides, polypeptides, polysaccharides, or polyvinyl pyrrolidones. Other non-biodegradable but durable polymers include without limitation ethylene-vinyl acetate co-polymer, polytetrafluoroethylene, polypropylene, polyethylene, and the like. Likewise, other suitable non-biodegradable polymers include without limitation silicones and polyurethanes.

In a further aspect, the polymer can be a poly(lactide), a poly(glycolide), a poly(lactide-co-glycolide), a poly(caprolactone), a poly(orthoester), a poly(phosphazene), a poly(hydroxybutyrate) or a copolymer containing a poly(hydroxybutarate), a poly(lactide-co-caprolactone), a polycarbonate, a polyesteramide, a polyanhydride, a poly(dioxanone), a poly(alkylene alkylate), a copolymer of polyethylene glycol and a polyorthoester, a biodegradable polyurethane, a poly(amino acid), a polyamide, a polyesteramide, a polyetherester, a polyacetal, a polycyanoacrylate, a poly(oxyethylene)/poly(oxypropylene) copolymer, polyacetals, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations, or blends thereof.

In a still further aspect, useful biocompatible polymers are those that comprise one or more residues of lactic acid, glycolic acid, lactide, glycolide, caprolactone, hydroxybutyrate, hydroxyvalerates, dioxanones, polyethylene glycol (PEG), polyethylene oxide, or a combination thereof. In a still further aspect, useful biocompatible polymers are those that comprise one or more residues of lactide, glycolide, caprolactone, or a combination thereof.

In one aspect, useful biocompatible polymers are those that comprise one or more blocks of hydrophilic or water soluble polymers, including, but not limited to, polyethylene glycol, (PEG), or polyvinyl pyrrolidone (PVP), in combination with one or more blocks another biocompabible or biodegradable polymer that comprises lactide, glycolide, caprolactone, or a combination thereof.

In specific aspects, the biocompatible polymer can comprise one or more lactide residues. The polymer can comprise any lactide residue, including all racemic and stereospecific forms of lactide, including, but not limited to, L-lactide, D-lactide, and D,L-lactide, or a mixture thereof. Useful polymers comprising lactide include, but are not limited to poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide polymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL- polymers. In addition to copolymers comprising glycolide and DL-lactide or L-lactide, copolymers of L-lactide and DL-lactide are commercially available. Homopolymers of lactide or glycolide are also commercially available.

When the biocompatible polymer is poly(lactide-co-glycolide), poly(lactide), or poly(glycolide), the amount of lactide and glycolide in the polymer can vary. In a further aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In a further aspect, the biodegradable polymer can be poly(lactide), 95:5 poly(lactide-co-glycolide) 85:15 poly(lactide-co-glycolide), 75:25 poly(lactide-co-glycolide), 65:35 poly(lactide-co-glycolide), or 50:50 poly(lactide-co-glycolide), where the ratios are mole ratios.

In a further aspect, the polymer can be a poly(caprolactone) or a poly(lactide-co-caprolactone). In one aspect, the polymer can be a poly(lactide-caprolactone), which, in various aspects, can be 95:5 poly(lactide-co-caprolactone), 85:15 poly(lactide-co-caprolactone), 75:25 poly(lactide-co- caprolactone), 65:35 poly(lactide-co- caprolactone), or 50:50 poly(lactide-co- caprolactone), where the ratios are mole ratios.

In one aspect, the flexible body composite can comprise a terpolymer. In one aspect, the terpolymers are those terpolymers disclosed in U.S. Patent Application Serial No. 12/269135, filed November 12, 2008, (U.S. Patent Publication No. 2009/0124535) which is incorporated herein by this reference for all of its teachings of terpolymers and is considered part of this disclosure.

It is understood that any combination of the aforementioned biocompatible polymers can be used, including, but not limited to, copolymers thereof, mixtures thereof, or blends thereof. Likewise, it is understood that when a residue of a biocompatible polymer is disclosed, any suitable polymer, copolymer, mixture, or blend, that comprises the disclosed residue, is also considered disclosed. To that end, when multiple residues are individually disclosed *(i.e.,* not in combination with another), it is understood that any combination of the individual residues can be used. Further, any of the above polymers can be processed (*e.g*., cross-linked to a desired level, to achieve a substantially flexible elongate body. An additional cross-linking agent can be used, and/or radical, cation, or anion cross-linking of the existing polymer can be used.

If desired, an adhesive, which can, in various aspect, be separate from the substantially flexible elongate body, or can be part of the substantially flexible elongate body itself, can be present on one or more surfaces of a disclosed flexible body composite, that will contact the implant device surface, or the tissue of fluid of the subject. The adhesive can be any desired adhesive. In certain aspects, the substantially flexible elongate body itself can comprise a tacky polymer, which functions as an adhesive to which an implant device, or tissue or fluid of the subject can adhere. Methods of making the above disclosed polymers tacky are known in the art. Addititives for example, can be added to provide a tacky polymer that can be adhesive. In one aspect, tacky polymers can be those that comprise a *T*_{g} of less than about room temperature, including those polymers disclosed above which have glass transition temperatures of less than about room temperature. Thus, in certain aspects, the resiliently expandable polymer can contact a disclosed implant device, tissue or fluid of a subject both elastically and adhesively, through the use of a tacky polymer. Other suitable adhesives, other than the polymer itself, include without limitation thermoplastics, glycoproteins, mucopolysaccharides, bioadhesives, carbohydrates, starches, dextrin, sugars, gelatin, epoxy, acrylics, rubber, silicones, polyurethanes, pressure sensitive adhesives, polyesters, polyethers, polychloroprene, natural gums, peroxides, silanes, isocyanates, or combinations, mixtures, and blends thereof.

In one aspect, the adhesive can be a biodegradable adhesive, including without limitation, poly(lactide-co-caprolactone), poly(glycolide-co-caprolactone), or combinations, mixtures, and blends thereof.

The adhesive can be applied to the first surface of the polymer through methods known in the art. Adhesives can be applied, for example, through spin-coating, drop-casting, brushing, or spraying an adhesive composition onto the first surface of the polymer.

As discussed above, the flexible body composite comprises a bioactive agent. The bioactive agent can be a releasable bioactive agent, *i.e.,* a bioactive agent that can be released from the substantially flexible elongate body. In certain aspects, the bioactive agent can be in or on the substantially flexible elongate body.

Also disclosed are kits comprising the flexible body composites. The kit can be comprised one or more disclosed flexible body composites, in a package. In one aspect, the kits can comprise a mixture of the same or different flexible body composites. For example, the kit can comprise several sets of flexible body composites, each having a different, or the same, size. Such a kit may be useful for point of use applications of the flexible body composites, wherein one kit, for example, can provide flexible body composites that are compatible in size with a number of different implant devices.

Also disclosed are implant devices comprising the flexible body composites. The term "device" is any formulation or article that is greater than 1 mm in length in at least one dimension of the device. The device can comprise a disclosed flexible body composite. In a further aspect, the device has one dimension that is from 1 mm to 50 mm, 1.2 mm to 45 mm, 1.4 mm to 42 mm, 1.6 mm to 40 mm, 1.8 mm to 38 mm, or 2.0 mm to 36 mm, 5.0 mm to 33 mm, or 10 mm to 30 mm. In a further aspect, the device has one dimension that is greater than 3 cm, even up to or greater than 10 cm, 20 cm, or even 30 cm.

In one aspect, the implant device comprises a disclosed flexible body composite contacting at least a portion of the implant device.

The implant device can comprise any shape, such as a rod, a fiber, a cylinder, a bead, a ribbon, a disc, a wafer, a free-formed shaped solid, or a variety of other shaped solids. The device can have any regular or irregular shape and can have any cross section like circular, rectangular, triangular, oval, and the like. In a further aspect, the device comprises a cylindrical shape, such as a typical shape of an implantable pump.

The implant can be comprised of any suitable material, such as a metal (*e.g.,* titanium), metal composite, organic material, polymeric, or even ceramic material. The surface of the implant can be any shaped surface, and may have a porous, beaded or meshed ingrowth surface, as can be present in certain implants.

The implant device can be any type of medical implant. The implant devices can include, for example, implants for drug delivery, including drug delivery pumps; orthopedic implants, including spinal implants, implants for osseointegration or bone repair; medical stents, including stents with inherent drug delivery capability; prosthetic implants, including breast implants, muscle implants, and the like; dental implants; ear implants, including cochlear implants and hearing devices; cardiac implants including pacemakers, catheters, etc.; space filling implants; bioelectric implants; neural implants; internal organ implants, including dialysis grafts; defribrillators; monitoring devices; recording devices; stimulators, including deep brain stimulators, nerve stimulators, bladder stimulators, and diaphragm stimulators; implantable identification devices and information chips; artificial organs; drug administering devices; implantable sensors/biosensors; screws, tubes, rods, plates, or artificial joints.

In a further aspect, the implant device can be at least one of a pump, pacemaker, defribrillator, or stimulator, including deep brain stimulators, nerve stimulators, bladder stimulators, and diaphragm stimulators.

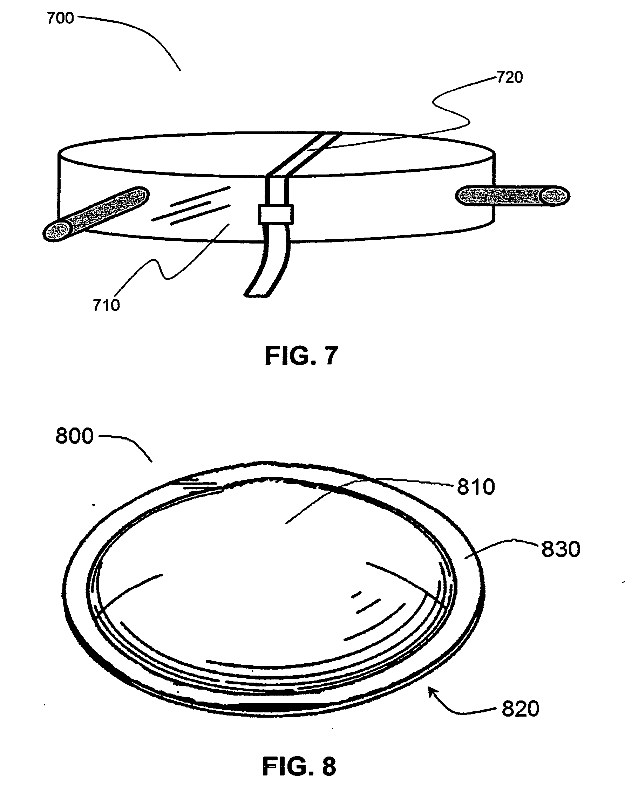

With reference to Figure 7, an implant device **700** can comprise a first implant device surface **710** that comprises a flexible body composite comprised of a substantially flexible elongate body **720,** which is contacting at least a portion, or all, of the device **700.** The device shown in the Figure 7 is an implantable pump.

Once the implant device is present in a subject, the substantially flexible elongate body can degrade, allowing the bioactive agent to be released in or near the tissue that is adjacent the implant site. If desired, a plurality of flexible body composites can be applied to the implant device.

Other implant devices that may benefit when used with the disclosed flexible body composites include those with one or more active surfaces, *e.g.,* a surface that enhances a connection between a tissue or fluid and the implant device, or a surface that allows for or enhances wound healing. The disclosed flexible body composites can be effective when applied to only a portion of the implant device, allowing for any active surface to remain exposed and functional when the implant device is implanted in a subject.

As discussed above, it can be desirable to deliver a bioactive agent at or near the tissue adjacent an implant site. The bioactive agent can help prevent some of the problems associated with implants, such as infection, or enhance the function of the implant itself. It can also be desirable to avoid pre-manufacturing an implant device with bioactive agent releasing capability, as discussed above. It should be appreciated that the composites, methods, and kits disclosed herein can allow for a point of use application of a flexible body composite onto the surface of an implant device, thus obviating the need to pre-manufacture implant devices having bioactive agent releasing capability.

In one aspect, a flexible body composite can be applied to an implant device surface close to or during the time of use. For example, a flexible body composite can be applied to an implant device by securing the flexible body composite onto the surface of the implant device, substantially close to the time when the implant device is implanted in a subject. In one aspect, the flexible body composite can be applied to an implant device in an operating suite, for example, by a physician or nurse.

The flexible body composite can be secured to the surface of the implant device prior to or after the time when the implant device is implanted in the subject. In one aspect, the implant device comprising the flexible body composite can be implanted into the subject. In a further aspect, the implant device can be implanted into the subject, and then the flexible body composite can be applied to the surface of the implant device. When implanting smaller implants, it may be beneficial to first secure the flexible body composite to the implant device surface before implanting the device in a subject.

In one aspect, the flexible body composite can be secured to the surface of the implant device on the same day (*i.e.,* within 24 hours) of the implant surgery, including, for example, within 23 hours, 20 hours, 15 hours, 10 hours, 5 hours, 3 hours, 2 hours, 1 hour, 30 minutes, 15 minutes, 10 minutes, 5 minutes, 2 minutes, 30 seconds, or during with the implant surgery itself.

If desired, the flexible body composite itself, with or without an implant device, can be implanted onto or in a tissue or fluid of a subject. In one aspect, the flexible body composite can be implanted onto or in a tissue or fluid that is near or adjacent to an implant site, *i.e.,* a site where an implant device has been implanted, or near or adjacent to a desired implant site.

Typically, before applying the flexible body composite to the implant device, the implant device surface can be cleaned or treated to remove any surface contaminants and to promote good adhesion of the substantially flexible elongate body. For example, the flexible body composite and/or the implant device can be sterilized *e.g.* by autoclaving under water steam. However, in some aspects, care may be needed to avoid irreversibility deforming the substantially flexible elongate body, or melting the substantially flexible elongate body. The flexible body composite or implant device comprising the flexible body composite can then be implanted into the subject using known surgical techniques. In certain aspects, it can be desirable to store the flexible body composites or kits comprising the composites in a sterilized container or package. In one aspect, the kit can comprise a sterilized package of the flexible body composites.

The disclosed methods can be used with any of the disclosed flexible body composites comprising a releasable bioactive agent. In one aspect, the method comprises securing the flexible body composite around at least a first surface of the implant device, or tissue or fluid of a subject. For example, the flexible body composite can be wrapped around the implant device, and the first portion of the flexible body composite can be connected to the second portion of the flexible body composite through the mechanical connection, or mechanical connection means, as discussed above. Optionally, an adhesive can be applied to a portion or the entire first surface of the substantially flexible elongate body, or to a portion or the entire implant device surface, to aid in securing the flexible body composite to the implant device.

The implant device can be implanted in any desired subject. The subject can be a vertebrate, such as a mammal, a fish, a bird, a reptile, or an amphibian. The subject of the herein disclosed methods can be, for example, a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered.

### iv) Adherent Biocompatible Terpolymer Compositions

In another aspect, disclosed are adherent biocompatible terpolymer compositions. In one aspect, the composition is a bioactive agent delivery composition comprising an adhering biocompatible terpolymer and a releasable bioactive agent. Also disclosed are kits comprising the disclosed compositions. Also disclosed are kits comprising 1) an adhering biocompatible terpolymer; and 2) a bioactive agent; wherein the terpolymer and bioactive agent are separated. Also disclosed are implant devices having a first implant device surface comprising a disclosed composition on at least a portion of the first implant device surface. Also disclosed are methods of applying a bioactive composition to an implant device, the method comprising applying disclosed composition onto a surface of an implant device, substantially close to the time when the implant device is implanted in a subject. Also disclosed are methods of applying a coating to an implant device, the method comprising applying a coating comprising the 1) adhering biocompatible terpolymer and 2) the bioactive agent of a disclosed kit onto a surface of the implant device. Also disclosed are methods for formulating a bioactive composition, the method comprising mixing an adhering biocompatible terpolymer and a bioactive agent, thereby forming the bioactive composition.

The terpolymer used with the compositions can be any suitable biocompatible terpolymer. In one aspect, the terpolymer is a biodegradable or non-biodegradable terpolymer. The polymers can be block or blocky ter- polymers, random ter- polymers, or star terpolymers. Any desired molecular weight terpolymer can be used, depending on the desired properties of the composition. In certain aspects, if a high strength composition is desired, then high molecular weight terpolymers can be used, for example, to meet strength requirements. In other aspects, low or medium molecular weight terpolymers can be used when, for example, when resorption time of the terpolymer, rather than material strength is desired.

The molecular weight of the terpolymer can be selected so as to provide a desired property of the composition. In certain aspects, the terpolymer can be provided by forming an adhering formulation of the terpolymer. In such aspects, the molecular weight should be high enough, or low enough, so that it forms satisfactory formulations. The molecular weight of a terpolymer is also important from the point of view that molecular weight influences the biodegradation rate of the terpolymer. For a diffusional mechanism of bioactive agent release, the terpolymer should remain intact until all of the drug is released from the terpolymer and then degrade. The drug can also be released from the terpolymer as the terpolymer bioerodes. By an appropriate selection of polymeric materials, a terpolymer formulation can be made such that the resulting biodegradable terpolymer exhibits both diffusional release and biodegradation release properties. Molecular weights can be measured by methods known in the art, including gel permeation chromatography, viscosity, light-scattering, among other methods.

The biodegrable terpolymer can be formulated so as to degrade within a desired time interval, once present in a subject. In some aspects, the time interval can be from about less than one day to about 1 month. Longer time intervals can extend to 6 months, including for example, terpolymer matrices that degrade from about ≥0 to about 6 months, or from about 1 to about 6 months. In other aspects, the terpolymer can degrade in longer time intervals, up to 2 years or longer, including, for example, from about ≥0 to about 2 years, or from about 1 month to about 2 years.

The desired bioactive agent release mechanism can influence the selection of the terpolymer. A composition can be selected so as to release or allow the release of a bioactive agent therefrom at a desired lapsed time after the composition has been implanted into a subject. In one aspect, the composition or terpolymer can be selected to release or allow the release of the bioactive agent prior to the bioactive agent beginning to diminish its activity, as the bioactive agent begins to diminish in activity, when the bioactive agent is partially diminished in activity, for example at least 25%, at least 50% or at least 75% diminished, when the bioactive agent is substantially diminished in activity, or when the bioactive agent is completely gone or no longer has activity.

In one aspect, the terpolymer can be a terpolymer that comprises one or more of polyesters, polyhydroxyalkanoates, polyhydroxybutyrates, polydioxanones, polyhydroxyvalerates, polyanhydrides, polyorthoesters, polyphosphazenes, polyphosphates, polyphosphoesters, polydioxanones, polyphosphoesters, polyphosphates, polyphosphonates, polyphosphates, polyhydroxyalkanoates, polycarbonates, polyalkylcarbonates, polyorthocarbonates, polyesteramides, polyamides, polyamines, polypeptides, polyurethanes, polyalkylene alkylates, polyalkylene oxalates, polyalkylene succinates, polyhydroxy fatty acids, polyacetals, polycyanoacrylates, polyketals, polyetheresters, polyethers, polyalkylene glycols, polyalkylene oxides, polyethylene glycols, polyethylene oxides, polypeptides, polysaccharides, or polyvinyl pyrrolidones. Other non-biodegradable but durable polymers include without limitation ethylene-vinyl acetate co-polymer, polytetrafluoroethylene, polypropylene, polyethylene, and the like. Likewise, other suitable non-biodegradable terpolymers include without limitation those that comprise silicones and polyurethanes.

In a further aspect, the terpolymer can be a terpolymer that comprises a poly(lactide), a poly(glycolide), a poly(lactide-co-glycolide), a poly(caprolactone), a poly(orthoester), a poly(phosphazene), a poly(hydroxybutyrate) or a copolymer containing a poly(hydroxybutarate), a poly(lactide-co-caprolactone), a polycarbonate, a polyesteramide, a polyanhydride, a poly(dioxanone), a poly(alkylene alkylate), a copolymer of polyethylene glycol and a polyorthoester, a biodegradable polyurethane, a poly(amino acid), a polyamide, a polyesteramide, a polyetherester, a polyacetal, a polycyanoacrylate, a poly(oxyethylene)/poly(oxypropylene) copolymer, polyacetals, polyketals, polyphosphoesters, polyhydroxyvalerates or a copolymer containing a polyhydroxyvalerate, polyalkylene oxalates, polyalkylene succinates, poly(maleic acid), and combinations, or blends thereof.

In a still further aspect, useful biodegradable terpolymers are those that comprise one or more residues of lactic acid, glycolic acid, lactide, glycolide, caprolactone, hydroxybutyrate, hydroxyvalerates, dioxanones, polyethylene glycol (PEG), polyethylene oxide, or a combination thereof. In a still further aspect, useful biodegradable terpolymers are those that comprise one or more residues of lactide, glycolide, caprolactone, or a combination thereof.

In one aspect, useful biodegradable terpolymers are those that comprise one or more blocks of hydrophilic or water soluble polymers, including, but not limited to, polyethylene glycol, (PEG), or polyvinyl pyrrolidone (PVP), in combination with one or more blocks another biocompabible or biodegradable terpolymer that comprises lactide, glycolide, caprolactone, or a combination thereof.

In specific aspects, the biodegradable terpolymer can comprise one or more lactide residues. The terpolymer can comprise any lactide residue, including all racemic and stereospecific forms of lactide, including, but not limited to, L-lactide, D-lactide, and D,L-lactide, or a mixture thereof. Useful terpolymers comprising lactide include, but are not limited to terpolymers comprising poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide terpolymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL- polymers. In addition to terpolymers comprising glycolide and DL-lactide or L-lactide, copolymers of L-lactide and DL-lactide are commercially available. Homopolymers of lactide or glycolide are also commercially available. These polymers can be combined to form a terpolymer

When the biodegradable terpolymer is terpolymer comprising poly(lactide-co-glycolide), poly(lactide), or poly(glycolide), the amount of lactide and glycolide in the terpolymer can vary. In a further aspect, the biodegradable terpolymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In a further aspect, the biodegradable terpolymer can be a terpolymer comprisnig poly(lactide), 95:5 poly(lactide-co-glycolide) 85:15 poly(lactide-co-glycolide), 75:25 poly(lactide-co-glycolide), 65:35 poly(lactide-co-glycolide), or 50:50 poly(lactide-co-glycolide), where the ratios are mole ratios.

In a further aspect, the terpolymer can be a a terpolymer comprising poly(caprolactone) or a poly(lactide-co-caprolactone). In one aspect, the terpolymer can be a terpolymer comprising poly(lactide-caprolactone), which, in various aspects, can be 95:5 poly(lactide-co-caprolactone), 85:15 poly(lactide-co-caprolactone), 75:25 poly(lactide-co-caprolactone), 65:35 poly(lactide-co- caprolactone), or 50:50 poly(lactide-co- caprolactone), where the ratios are mole ratios.

In a further aspect, the terpolymer can comprise at least one of, or all of, lactide (D or L), glycolide, or caprolactone. Any two of lactide (D or L), glycolide, or caprolactone, when present, can be present in any desired ratio, with respect to the other. In one aspect, the terpolymer can be a poly(lactide-co-glycolide-co-caprolactone). In a further aspect, the terpolymer can be a poly(D-lactide-co-glycolide-co-caprolactone). The ratio of lactide to glycolide to caprolactone can be any desired ratio. In one aspect, the ratio of lactide to glycolide to caprolactone is 20:30:50. In one aspect, the terpolymer is a poly(D-lactide-co-glycolide-co-caprolactone) with at least one ester end cap, wherein the ratio of lactide to glycolide to caprolactone is 20:30:50, which has a viscosity of about 0.1 dL/g (abbreviated as 20:30:50 DLGCL 1E).

In a further aspect, the terpolymer can be a terpolymer comprising an optionally endcapped polyethylene glycol. Examples of optionally endcapped polyethylene glycol include without limitation, polyethylene glycol (not endcapped), and mPEG, which is methoxypoly(ethylene glycol), among others. In one aspect, the terpolymer is a tacky terpolymer, such as, for example, a poly(D-lactide-co-glycolide-co-mPEG). In a further aspect, the terpolymer is a poly(D-lactide-co-glycolide-co-mPEG) wherein the ratio of lactide to glycolide is 50:50, and wherein the molecular weight of mPEG is about 2000 Daltons. Such a terpolymer can have an ester endcap and a viscosity of about 0.2 dL/g (abbreviated as 50:50 DLG mPEG 2000 2E).

In one aspect, the terpolymer comprises at least one of lactide, glycolide, caprolactone, optionally endcapped polyethylene glycol (PEG), or a combination thereof. In a further aspect, the terpolymer comprises a terpolymer of lactide, glycolide, and caprolactone residues, wherein the terpolymer comprises an end group that is a residue of an initiator and wherein the initiator is a non-crystalline primary or secondary alcohol. In a still further aspect, the terpolymer comprises at least one of poly(lactide-co-glycolide-co-optionally endcapped PEG), or poly(lactide-co-glycolide-co-caprolactone, or a combination thereof.

In one aspect, the terpolymers are those terpolymers disclosed in U.S. Patent Publication No. 2009/0124535.

It is understood that any combination of the aforementioned biodegradable terpolymers can be used, including, but not limited to, mixtures thereof, or blends thereof. Likewise, it is understood that when a residue of a biodegradable terpolymer is disclosed, any suitable terpolymer, mixture, or blend, that comprises the disclosed residue, is also considered disclosed. To that end, when multiple residues are individually disclosed (*i*.*e*., not in combination with another), it is understood that any combination of the individual residues can be used.

In one aspect, the terpolymer can be an adhering terpolymer that is capable of sticking to a contacting surface. In certain aspects, the terpolymer itself can be a tacky terpolymer, which functions as an adhesive to which a surface can directly adhere. Methods of making the above disclosed polymers tacky are known in the art. Addititives for example, can be added to provide a tacky terpolymer that can be adhesive. In one aspect, tacky polymers can be those that comprise a *T*_{g} of less than about room temperature, including those polymers disclosed above which have glass transition temperatures of less than about room temperature. In other aspects, the terpolymer can be a gel or gel-like, wax or wax-like, VASELINE® like, viscous, tacky, or a combination thereof.

As discussed above, the composition comprises a bioactive agent. The bioactive agent can be a releasable bioactive agent, *i*.*e*., a bioactive agent that can be released from the terpolymer. In certain aspects, the bioactive agent can be in or on the terpolymer.

In one aspect, the terpolymer can be stored separately from the bioactive agent, and the composition can be formulated near or at the time of use. Such aspects allow for the drug stability to remain independent, and/or free from contamination or alteration due to the presence of the terpolymer. It should also be appreciated that many of those biodegradable, tacky terpolymers are well characterized and generally accepted by the FDA for parenteral use.

Also disclosed are kits comprising the compositions. A kit can have various compositions that can be same or different depending on their intended use, all packaged together in one unit.

In a further aspect, a kit comprises 1) an adhering biocompatible terpolymer; and 2) a bioactive agent; wherein the terpolymer and bioactive agent are separated. In one aspect, the terpolymer is present in a first container, and the bioactive agent is present in a second container. In a further aspect, the terpolymer is present in a first dispensing system, and the bioactive agent is present in a second dispensing system. The first dispensing system can dispense the terpolymer, while the second dispensing system can dispense the bioactive agent, such that a mixture is formed that comprises the terpolymer and the bioactive agent. Such a process can be conveniently carried out, in various aspects, using a syringe as the first and/or second dispensing system.

The compositions, as discussed above in various aspects, can be provided by admixing the adhering biocompatible terpolymer and the bioactive agent to form a composition. In one aspect, the terpolymer and the bioactive agent can be stored separately until close to, or at, the time of use. For example, the terpolymer and the bioactive agent can be stored separately in a disclosed kit.

In one aspect, a method for formulating a bioactive composition comprises mixing the adhering biocompatible terpolymer and the bioactive agent, thereby forming the bioactive composition. The mixing step can be performed at any time. In one aspect, the mixing step is performed close to, or at, the time of use, or close to, or at, the time of implant surgery, including without limitation on the same day *(i.e.,* within 24 hours) of the implant surgery, including, for example, within 23 hours, 20 hours, 15 hours, 10 hours, 5 hours, 3 hours, 2 hours, 1 hour, 30 minutes, 15 minutes, 10 minutes, 5 minutes, 2 minutes, 30 seconds, or during the implant surgery itself.

Also disclosed are implant devices comprising the compositions. The device can be any formulation or article that is greater than 1 mm in length in at least one dimension of the device. The device can comprise a disclosed composition. In a further aspect, the device has one dimension that is from 1 mm to 50 mm, 1.2 mm to 45 mm, 1.4 mm to 42 mm, 1.6 mm to 40 mm, 1.8 mm to 38 mm, or 2.0 mm to 36 mm, 5.0 mm to 33 mm, or 10 mm to 30 mm. In a further aspect, the device has one dimension that is greater than 3 cm, even up to or greater than 10 cm, 20 cm, or even 30 cm.

In one aspect, the implant device comprises at least a first implant device surface comprising a composition on at least a portion thereof, wherein if the composition has a length dimension substantially larger than a width dimension and a width dimension substantially larger than a thickness dimension, then the width dimension is less than about 2 mm.

The implant device can comprise any shape, such as a rod, a fiber, a cylinder, a bead, a ribbon, a disc, a wafer, a free-formed shaped solid, or a variety of other shaped solids. The device can have any regular or irregular shape and can have any cross section like circular, rectangular, triangular, oval, and the like. In a further aspect, the device comprises a cylindrical shape, such as a typical shape of an implantable pump.

The implant can be comprised of any suitable material, such as a metal (*e.g.,* titanium), metal composite, organic material, polymeric, or even ceramic material. The surface of the implant can be any shaped surface, and may have a porous, beaded or meshed ingrowth surface, as can be present in certain implants.

The implant device can be any type of medical implant. The implant devices can include, for example, implants for drug delivery, including drug delivery pumps; orthopedic implants, including spinal implants, implants for osseointegration or bone repair; medical stents, including stents with inherent drug delivery capability; prosthetic implants, including breast implants, muscle implants, and the like; dental implants; ear implants, including cochlear implants and hearing devices; cardiac implants including pacemakers, catheters, etc.; space filling implants; bioelectric implants; neural implants; internal organ implants, including dialysis grafts; defribrillators; monitoring devices; recording devices; stimulators, including deep brain stimulators, nerve stimulators, bladder stimulators, and diaphragm stimulators; implantable identification devices and information chips; artificial organs; drug administering devices; implantable sensors/biosensors; screws; tubes; rods; plates; or artificial joints.

In a further aspect, the implant device can be at least one of a pump, pacemaker, defribrillator, or stimulator, including deep brain stimulators, nerve stimulators, bladder stimulators, and diaphragm stimulators.

Other implant devices that may benefit when used with the disclosed compositions include those with one or more active surfaces, *e.g.,* a surface that enhances a connection between a tissue or fluid and the implant device, or a surface that allows for or enhances wound healing. The disclosed compositions can be effective when applied to only a portion of the implant device, allowing for any active surface to remain exposed and functional when the implant device is implanted in a subject.

As discussed above, it can be desirable to deliver a bioactive agent at or near the tissue adjacent an implant site. The bioactive agent can help prevent some of the problems associated with implants, such as infection, or enhance the function of the implant itself. It can also be desirable to avoid pre-manufacturing an implant device with bioactive agent releasing capability, as discussed above. It should be appreciated that the compositions, methods, and kits disclosed herein can allow for a point of use application of a composition onto the surface of an implant device, thus obviating the need to pre-manufacture implant devices having bioactive agent releasing capability.

In one aspect, a composition or coating (*e*.*g*., a coating wherein the terpolymer and bioactive agent are separated) can be applied to an implant device surface close to or during the time of use. For example, a composition can be applied to an implant device by applying (*e.g.* rubbing, brushing, smearing, dispensing from a dispensing system or kit, etc.) the composition or coating onto the surface of the implant device, substantially close to the time when the implant device is implanted in a subject. In one aspect, the composition or coating can be applied to an implant device in an operating suite, for example, by a physician or nurse.

In a further aspect, an implant device can be coated with a coating comprising the 1) adhering biocompatible terpolymer and 2) the bioactive agent of a disclosed kit onto a surface of the implant device. In one aspect, the 1) adhering biocompatible terpolymer and 2) the bioactive agent are mixed prior to, at, or after the coating is applied to the surface of the implant device. Thus, the term "coating" includes without limitation at least two coatings, wherein the at least two coatings are separated from each other, on the device surface. For example, a first region of the device surface can comprise the terpolymer, and a second region of the device surface can comprise the bioactive agent, which is separated from the terpolymer. Such a coating can be formed into a disclosed composition by mixing the terpolymer and the bioactive agent, when they are present on the device surface. When present separately on a device surface, the terpolymer and the bioactive agent can be mixed using known methods, for example, by smearing the two coatings together with an applicator or q-tip. In a further aspect, the terpolymer and the bioactive agent are mixed. In a still further aspect, the terpolymer and the bioactive agent are first mixed and then applied, as a composition, to the implant device surface.

When the terpolymer and the bioactive agent are stored or kept separately from each other until the time of use, the terpolymer and bioactive agent can be mixed at any desired time. In one aspect, the adhering biocompatible terpolymer and the bioactive agent are mixed prior to applying the coating to the device surface. Thus, in this aspect, a composition comprising the terpolymer and the bioactive agent is first formed, prior to applying the coating to the device surface. In a further aspect, as discussed above, the terpolymer and the bioactive agent are mixed after each are individually and separately present on the device surface. In a still further aspect, the terpolymer and the bioactive agent are mixed at the same time, or a time close to the time, that each are individually being applied to the device surface.

The compositions and coatings can be applied to the surface of the implant device prior to or after the time when the implant device is implanted in the subject. In one aspect, the implant device comprising the composition and/or coatings can be implanted into the subject. In a further aspect, the implant device can be implanted into the subject, and then the composition and/or coating can be applied to the surface of the implant device. When implanting smaller implants, it may be beneficial to first apply the composition and/or coating to the implant device surface.

In one aspect, the composition and/or coating can be applied to the surface of the implant device on the same day (*i.e.,* within 24 hours) of the implant surgery, including, for example, within 23 hours, 20 hours, 15 hours, 10 hours, 5 hours, 3 hours, 2 hours, 1 hour, 30 minutes, 15 minutes, 10 minutes, 5 minutes, 2 minutes, 30 seconds, or during the implant surgery itself.

If desired, the composition and/or coating itself, with or without an implant device, can be implanted onto or in a tissue or fluid of a subject. In one aspect, the composition and/or coating can be implanted onto or in a tissue or fluid that is near or adjacent to an implant site, *i.e.,* a site where an implant device has been implanted, or near or adjacent to a desired implant site.

Typically, before applying the composition and/or coating to the implant device, the implant device surface can be cleaned or treated to remove any surface contaminants and to promote good adhesion of the terpolymer. For example, the composition, coating and/or the implant device can be sterilized. The composition and/or coating, or implant device comprising the composition and/or coating can then be implanted into the subject using known surgical techniques. In certain aspects, it can be desirable to store the compositions, components thereof, or kits comprising the compositions, or components thereof, in a sterilized container or package. In one aspect, the kit can comprise a sterilized package of the compositions, or components thereof, as discussed above.

In one aspect, the disclosed methods can be used with compositions comprising a releasable bioactive agent. In a further aspect, the methods can be used with compositions comprising an adhering biocompatible terpolymer and a bioactive agent. The composition can be applied to the surface of the implant, or to the tissue or fluid of the subject, by contacting the surface of the implant with the terpolymer composition, or component thereof.

The implant device can be implanted in any desired subject. The subject can be a vertebrate, such as a mammal, a fish, a bird, a reptile, or an amphibian. The subject of the herein disclosed methods can be, for example, a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered.

### Example iv.1 (Prophetic)

In a first example, a terpolymer composition is provided. A first syringe contains a viscous terpolymer such as 20:30:50 DLGCL 1E. A second syringe contains the bioactive agent, which in this case is an antibiotic, dissolved or suspended in a polymer compatible solvent (DMSO, NMP, etc). Prior to applying the composition to an implant device surface, the two syringes can be connected, and the components can be mixed by pushing the syringe plungers back and forth. The bioactive agent content can range from 0.1 wt% up to approximately 60 wt%, or greater, depending on the desired dose. The resulting viscous solution/suspension can be directly applied to the implant device surface with the syringe. A controlled release of the active agent can be achieved with a duration ranging from hours to days, as discuused above.

### Example iv.2 (Prophetic)

In a second example, another terpolymer composition is provided. A first syringe contains a viscous terpolymer such as 20:30:50 DLGCL 1E. A second syringe contains the bioactive agent in the form of a dry powder. The two syringes are connected and the active agent is suspended in the terpolymer by pushing the syringe plungers back and forth. The resulting viscous suspension can be directly applied to an implant device surface with the syringe. Similarly, the drug content can range from 0.1 wt% up to approximately 60 wt%, or greater as needed to control dose and release rate.

### Example iv.3 (Prophetic)

In a third example, a single syringe method for providing a terpolymer composition is disclosed. The terpolymer, active agent, and cosolvent (if desired or necessary) can be combined together and packaged in a single syringe. The drug content can range from 0.1 wt% up to about 60 wt%, or greater, depending on the desired dose. The resulting viscous solution/suspension can be directly applied to the surface of an implant device with the syringe. A controlled release of the active agent can be achieved with a duration ranging from hours to days.

### Example iv.4 (Prophetic)

In a fourth example, a single packing system comprising a terpolymer composition is provided. A single syringe or other suitable packaging device contains a tacky terpolymer such as 50:50 DLG mPEG 2000 2E. A second syringe or packaging device contains the bioactive agent in the form of a dry powder or film. The tacky terpolymer can be applied and spread onto the surface of the implant device, thus providing an adhesive surface to which the bioactive agent formulation can be applied. The bioactive agent formulation can then be applied to the tacky terpolymer surface. A controlled release of the bioactive agent can be achieved with a duration ranging from hours to days.

### Example iv.5 (Prophetic)

In a fifth example, another terpolymer composition is provided. A single syringe system or other suitable packaging device comprises the terpolymer such as 50:50 DLG mPEG 2K, the bioactive agent, and cosolvent (if desired or necessary). The bioactive agent content can range from 0.1 wt% up to about 60 wt%, or greater, depending on the desired dose. The resulting viscous solution/suspension can be directly applied to an implant device surface with the syringe and spread onto the surface of the device. A controlled release of the active agent can be achieved with a duration ranging from hours to days.

### v) Implantable Suction Cup Composites

Also described herein are suction cup composites that can be applied to an implant device, or to a tissue or fluid of a subject. The suction cup composites can release a bioactive agent into the subject. The composites described herein allow for controlled-release, extended-release, modified-release, sustained-release, pulsatile-release, delayed-release, or programmed-release of the bioactive agent.

In one aspect, the suction cup composite comprises a cup member defining a cavity and having a substantially planar rim, wherein the cup member comprises a resilient biocompatible polymer; wherein the resilient biocompatible polymer comprises a bioactive agent disposed therein the polymer. In one aspect, the rim is substantially planar. In a further aspect, the rim becomes substantially planar when contacting a surface. Thus, in certain aspects, the rim is substantially non-planar, but is capable of becoming planar when contacting a surface.

With reference to Figure 8, the cup member **810** of an exemplary suction cup composite **800** comprises at least one convex surface, as shown, such that the cup member defines a cavity **820** that opposes the convex surface. To enable the cup member to adhere to a contacting surface, the rim **830** of the cup member is substantially planar. In one aspect, the disclosed suction cup composite functions as a suction cup. In one aspect, the convex surface may need to only be slightly convex, or have a slight curvature.

The suction cup composites can have any desired size. In general, the size selection of the suction cup composite can be influenced by the desired loading of the bioactive agent. Generally, the more bioactive agent that is desired, the larger the suction cup composite will be. The size can also be selected so as to provide the desired release properties of the suction cup composite. In addition, when the suction cup composite is applied to an implant device, the size of the implant device can be of importance when selecting the size of the suction cup composite. For example, it can be desirable for portions of the implant device surface to remain exposed. In these instances, the size of the implant can be selected so as to not completely cover the implant device surface.

For example, the suction cup composites can have diameters (the greatest distance across the cavity defined by the cup member) including without limitation of from about 1 cm to about 50 cm or greater, from about 5 cm to about 25 cm, or from about 7 cm to about 15 cm, including those suction cup composites comprising cavities having a diameter of about 2, 3, 4, 5, 6, 7, 8, 9, 10, 11, 12, 13, 14, 15, 16, 17, 18, 19, 20, 30, 40, or 45 cm, or larger. Additionally, the suction cup composites can have diameters of less than about 1 cm, including for example, from about 0.1 microns to about 10 mm, or from about 0.1 microns to about 1 cm. Likewise, the suction cup composites can have any desired cavity depth, which can, in various aspects, depend on the desired adhesion strength of the suction cup composite once applied to a contacting surface. In one aspect, the suction cup composites can have a cavity depth (*i.e.,* the greatest cavity depth) of from about 0.1 microns to about 500 cm or greater, from about 5 microns to about 400 cm, from about 20 microns to about 200 cm, or from about 50 microns to about 100 cm, including those suction cup composites having cavity depths of about 0.1 microns, 1 micron, 50 microns, 100 microns, 200 microns, 1 mm, 5 mm, 10 mm, 20 mm, 50 mm, 1 cm, 2 cm, 3 cm, 4 cm, 5 cm, 8 cm, 10 cm, 20 cm, 30 cm, 40 cm, 50 cm, 60 cm, 70 cm, 80 cm, 90 cm, 100 cm, 150 cm, 200 cm, 250 cm, 300 cm, 350 cm, 400 cm, 450 cm, or 500 cm or greater.

The cup member can also have any desired thickness. In one aspect, the cup member can be as thin as a thin film, for example, having a thickness of from about 50 nm (or less) to about 1000 nm, or greater. In other aspects, the cup member can have a larger thickess, including without limitation thicknesses from about 1000 nm to about 500 cm, or from about 5 cm to about 100 cm, or from about 10 cm to about 80 cm, or from about 20 cm to about 50 cm, or larger.

The cup member can have any desired shape. In one aspect, the planar rim has a shape that is substantially circular, substantially rectangular, or substantially elliptical (oval-like).

In one aspect, the cup member can comprise a polymer with a mechanical property which enables the cup member to be deformed when contacting a contact surface. The polymer should be at least partially resilient, so as to be capable of recovering from deformation to form a seal that adheres the suction cup composite to the contacting surface.

In one aspect, the polymer can be an elastomer. Elastic polymers are those that can undergo a reversible elongation at a relatively low stress. Such polymers include without limitation amorphous polymers, polymers with low glass transition temperatures (*T*_{g}), polymers with high polymer chain mobility, cross-linked polymers, or a combination thereof. In one aspect, suitable polymers are those that are substantially amorphous and have a low *T*_{g}. Suitable glass transition temperatures include, without limitation, about 25 °C or less, about 15 °C or less, about 10 °C or less, about 0 °C or less, about -10 °C or less, about -20 °C or less, about -30 °C or less, about -40 °C or less, about -50 °C or less, about - 60 °C or less, about -70 °C or less, or less than -70 °C. In a further aspect, suitable polymers are those that are cross-linked. The degree of cross-linking for an elastic polymer can be typically selected based on the desired resilience of the cup member. Generally, at least some degree of cross-linking can be useful to achieve relatively rapid deformation and nearly complete reversibility, effective to form a seal on a contacting surface.

Suitable elastic polymers can have a relatively low initial force modulus (e.g., less than 100 N/cm), so that the polymer can be expanded without an inconvenient amount of force. Typically, however, the force modulus should increase beyond the initial force modulus upon expansion of the polymer. In certain aspects, to maintain high strength at a high expansive force, a polymer can be cross-linked, as discussed above, and/or can comprise biocompatible filler materials which add strength to the. Additionally, the polymer can be engineered to undergo a small amount of crystallization at high elongation, which can add strength to the polymer during forceful expansion.

In a further aspect, the cup member comprises a less reslient polymer, such as a semi-rigid polymer, or a polymer that is not an elastomer, but also not as rigid as a hard fiber. To that end, in some aspects, the cup member should be flexible enough to allow for the suction cup composite to be deformed and secured to a contacting surface. In one aspect, the cup member can be a plastic, such as a resilient, flexible plastic. Suitable flexible plastics are those that exhibit moderate to high ranges of crystallinity. Typically, a flexible plastic has a force modulus of from about 10,000 N/cm² to about 400,000 N/cm², with tensile strengths of from about 1000 N/cm² to about 10,000 N/cm². In one aspect, a flexible plastic can have an ultimate elongation percentage of from about 10% to about 1000%. For example, polyethylene is a typical flexible plastic with a tensile strength of about 2500 N/cm², a force modulus of about 20,000 N/cm², and an ultimate elongation of about 500 %. Other typical flexible plastics include without limitation polypropene and poly(hexamethylene adipamide). Additionally, those polymers discussed below can be engineered (e.g., cross-linked, or processed with additives) to provide the desired mechanical properties of the suction cup composite, for example, flexibility and rigidity.

The cup member used with the suction cup composites can comprise any biocompatible, biodegradable, or non-biodegradable polymer. The polymers disclosed herein can be homopolymers or copolymers. The polymers can be block or blocky co- or ter- polymers, random co- or ter- polymers, star polymers, or dendrimers. Any desired molecular weight polymer can be used, depending on the desired properties of the suction cup composite. In certain aspects, if a high strength suction cup composite is desired, then high molecular weight polymers can be used, for example, to meet strength requirements. In other aspects, low or medium molecular weight polymers can be used when, for example, when resorption time of the polymer, rather than material strength is desired. In one aspect, the polymer can be present as a blend of two or more polymers.

The molecular weight of the polymer can be selected so as to provide a desired property of the suction cup composite. In certain aspects, the suction cup composite can be provided by forming a molded composite of the polymer. In such aspects, the molecular weight should be such to allow a sufficient molded composite to form. The molecular weight should also be suitable to allow the suction cup composite to be resiliently expanded. The molecular weight of a polymer is also important from the point of view that molecular weight influences the biodegradation rate of the polymer. For a diffusional mechanism of bioactive agent release, the polymer should remain intact until all of the drug is released from the polymer and then degrade. The drug can also be released from the polymer as the polymer bioerodes. By an appropriate selection of polymeric materials a polymer formulation can be made such that the resulting biodegradable polymer exhibits both diffusional release and biodegradation release properties. Molecular weights can be measured by methods known in the art, including gel permeation chromatography, viscosity, light scattering, among other methods.

The suction cup composite can be formulated so as to degrade within a desired time interval, once present in a subject. In some aspects, the time interval can be from about less than one day to about 1 month. Longer time intervals can extend to 6 months, including for example, polymer matrices that degrade from about ≥0 to about 6 months, or from about 1 to about 6 months. In other aspects, the polymer can degrade in longer time intervals, up to 2 years or longer, including, for example, from about ≥0 to about 2 years, or from about 1 month to about 2 years.

The desired bioactive agent release mechanism can influence the selection of the polymer. A biodegradable polymer can be selected so as to release or allow the release of a bioactive agent therefrom at a desired lapsed time after the suction cup composite has been implanted into a subject. For example, the polymer can be selected to release or allow the release of the bioactive agent prior to the bioactive agent beginning to diminish its activity, as the bioactive agent begins to diminish in activity, when the bioactive agent is partially diminished in activity, for example at least 25%, at least 50% or at least 75% diminished, when the bioactive agent is substantially diminished in activity, or when the bioactive agent is completely gone or no longer has activity.

In one aspect, the polymer can be one or more of polyesters, polyhydroxyalkanoates, polyhydroxybutyrates, polydioxanones, polyhydroxyvalerates, polyanhydrides, polyorthoesters, polyphosphazenes, polyphosphates, polyphosphoesters, polydioxanones, polyphosphoesters, polyphosphates, polyphosphonates, polyphosphates, polyhydroxyalkanoates, polycarbonates, polyalkylcarbonates, polyorthocarbonates, polyesteramides, polyamides, polyamines, polypeptides, polyurethanes, polyalkylene alkylates, polyalkylene oxalates, polyalkylene succinates, polyhydroxy fatty acids, polyacetals, polycyanoacrylates, polyketals, polyetheresters, polyethers, polyalkylene glycols, polyalkylene oxides, polyethylene glycols, polyethylene oxides, polypeptides, polysaccharides, or polyvinyl pyrrolidones. Other non-biodegradable but durable polymers include without limitation ethylene-vinyl acetate co-polymer, polytetrafluoroethylene, polypropylene, polyethylene, and the like. Likewise, other suitable non-biodegradable polymers include without limitation silicones and polyurethanes.

In a further aspect, the polymer can be a poly(lactide), a poly(glycolide), a poly(lactide-co-glycolide), a poly(caprolactone), a poly(orthoester), a poly(phosphazene), a poly(hydroxybutyrate) or a copolymer containing a poly(hydroxybutarate), a poly(lactide-co-caprolactone), a polycarbonate, a polyesteramide, a polyanhydride, a poly(dioxanone), a poly(alkylene alkylate), a copolymer of polyethylene glycol and a polyorthoester, a biodegradable polyurethane, a poly(amino acid), a polyamide, a polyesteramide, a polyetherester, a polyacetal, a polycyanoacrylate, a poly(oxyethylene)/poly(oxypropylene) copolymer, polyacetals, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations, or blends thereof.

In a still further aspect, useful biodegradable polymers are those that comprise one or more residues of lactic acid, glycolic acid, lactide, glycolide, caprolactone, hydroxybutyrate, hydroxyvalerates, dioxanones, polyethylene glycol (PEG), polyethylene oxide, or a combination thereof. In a still further aspect, useful biodegradable polymers are those that comprise one or more residues of lactide, glycolide, caprolactone, or a combination thereof.

In one aspect, useful biodegradable polymers are those that comprise one or more blocks of hydrophilic or water soluble polymers, including, but not limited to, polyethylene glycol, (PEG), or polyvinyl pyrrolidone (PVP), in combination with one or more blocks another biocompabible or biodegradable polymer that comprises lactide, glycolide, caprolactone, or a combination thereof.

In specific aspects, the biodegradable polymer can comprise one or more lactide residues. The polymer can comprise any lactide residue, including all racemic and stereospecific forms of lactide, including, but not limited to, L-lactide, D-lactide, and D,L-lactide, or a mixture thereof. Useful polymers comprising lactide include, but are not limited to poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide polymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL- polymers. In addition to copolymers comprising glycolide and DL-lactide or L-lactide, copolymers of L-lactide and DL-lactide are commercially available. Homopolymers of lactide or glycolide are also commercially available.

When the biodegradable polymer is poly(lactide-co-glycolide), poly(lactide), or poly(glycolide), the amount of lactide and glycolide in the polymer can vary. In a further aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In a further aspect, the biodegradable polymer can be poly(lactide), 95:5 poly(lactide-co-glycolide) 85:15 poly(lactide-co-glycolide), 75:25 poly(lactide-co-glycolide), 65:35 poly(lactide-co-glycolide), or 50:50 poly(lactide-co-glycolide), where the ratios are mole ratios.

In a further aspect, the polymer can be a poly(caprolactone) or a poly(lactide-co-caprolactone). In one aspect, the polymer can be a poly(lactide-caprolactone), which, in various aspects, can be 95:5 poly(lactide-co-caprolactone), 85:15 poly(lactide-co-caprolactone), 75:25 poly(lactide-co- caprolactone), 65:35 poly(lactide-co- caprolactone), or 50:50 poly(lactide-co- caprolactone), where the ratios are mole ratios.

In one aspect, the suction cup composite can comprise a terpolymer. In one aspect, the terpolymers are those terpolymers disclosed in U.S. Patent Application Serial No. 12/269135, filed November 12, 2008, (U.S. Patent Publication No. 2009/0124535) which is incorporated herein by this reference for all of its teachings of terpolymers and is considered part of this disclosure.

It is understood that any combination of the aforementioned biodegradable polymers can be used, including, but not limited to, copolymers thereof, mixtures thereof, or blends thereof. Likewise, it is understood that when a residue of a biodegradable polymer is disclosed, any suitable polymer, copolymer, mixture, or blend, that comprises the disclosed residue, is also considered disclosed. To that end, when multiple residues are individually disclosed (*i.e*., not in combination with another), it is understood that any combination of the individual residues can be used. Further, any of the above polymers can be processed (*e.g*., cross-linked to a desired level, to achieve a resilient cup member. An additional cross-linking agent can be used, and/or radical, cation, or anion cross-linking of the existing polymer can be used.

If desired, an adhesive, which can, in various aspect, be separate from the cup member, or can be part of the cup member itself, can be present on the contacting rim of a disclosed suction cup composite, that will contact the implant device surface, or the tissue of fluid of the subject. The adhesive can be any desired adhesive. In certain aspects, the cup member itself can comprise a tacky polymer, which functions as an adhesive to which an implant device, or tissue or fluid of the subject can adhere. Methods of making the above disclosed polymers tacky are known in the art. Addititives for example, can be added to provide a tacky polymer that can be adhesive. In one aspect, tacky polymers can be those that comprise a *T_{g}* of less than about room temperature, including those polymers disclosed above which have glass transition temperatures of less than about room temperature. Thus, in certain aspects, the resiliently expandable polymer can contact a disclosed implant device, tissue or fluid of a subject both elastically and adhesively, through the use of a tacky polymer. Other suitable adhesives, other than the polymer itself, include without limitation thermoplastics, glycoproteins, mucopolysaccharides, bioadhesives, carbohydrates, starches, dextrin, sugars, gelatin, epoxy, acrylics, rubber, silicones, polyurethanes, pressure sensitive adhesives, polyesters, polyethers, polychloroprene, natural gums, peroxides, silanes, isocyanates, or combinations, mixtures, and blends thereof.

In one aspect, the adhesive can be a biodegradable adhesive, including without limitation, poly(lactide-co-caprolactone), poly(glycolide-co-caprolactone), or combinations, mixtures, and blends thereof.

The adhesive can be applied to the rim of the cup member through methods known in the art. Adhesives can be applied, for example, through spin-coating, drop-casting, brushing, or spraying an adhesive composition onto the first surface of the polymer.

In other aspects, the suction cup composite does not comprise an adhesive. In a further aspect, the rim of the suction cup composite does not comprise an adhesive. It will be apparent that the disclosed suction cup composites can be sealed to a contacting surface without the use of an adhesive.

In one aspect, the rim of the cup member can be moistened, or wetted to enhance the adhesion of the cup member to the contacting surface. Thus, in one aspect, the suction cup composite comprises a moistened rim.

In a further aspect, the cup member can comprise an opacifier. For this aspect, any desired opacifier can be used. An opacifier can be useful to view the suction cup composite or the device comprising same in the subject, through an opacifier signal, such as fluorescence, or for example, through x-ray among other diagnostic techniques. In one aspect, the opacifier can be without limitation barium sulfate, which can, in various aspects, make an implant device visible or more visible by an imaging technique, such as, for example, x-ray, among others.

As discussed above, the suction cup composite comprises a bioactive agent. The bioactive agent can be a releasable bioactive agent, *i.e*., a bioactive agent that can be released from the suction cup composite. In certain aspects, the bioactive agent can be in or on the cup member. In a further aspect, the bioactive agent can be dispersed in the polymer.

Also disclosed are kits comprising the suction cup composites. The kit can be comprised one or more disclosed suction cup composites, in a package. In one aspect, the kits can comprise a mixture of the same or different suction cup composites. For example, the kit can comprise several sets of suction cup composites, each having a different or the same size. Such a kit may be useful for point of use applications of the suction cup composites, wherein one kit, for example, can provide suction cup composites that are compatible in size with a number of different implant devices.

Also disclosed are implant devices comprising the suction cup composites. The term "device" is any formulation or article that is greater than 1 mm in length in at least one dimension of the device. The device can comprise a disclosed suction cup composite. In a further aspect, the device has one dimension that is from 1 mm to 50 mm, 1.2 mm to 45 mm, 1.4 mm to 42 mm, 1.6 mm to 40 mm, 1.8 mm to 38 mm, or 2.0 mm to 36 mm, 5.0 mm to 33 mm, or 10 mm to 30 mm. In a further aspect, the device has one dimension that is greater than 3 cm, even up to or greater than 10 cm, 20 cm, or even 30 cm.

In one aspect, the implant device comprises a disclosed suction cup composite contacting at least a portion of the implant device.

The implant device can comprise any shape, such as a rod, a fiber, a cylinder, a bead, a ribbon, a disc, a wafer, a free-formed shaped solid, or a variety of other shaped solids. The device can have any regular or irregular shape and can have any cross section like circular, rectangular, triangular, oval, and the like. In a further aspect, the device comprises a cylindrical shape, such as a typical shape of an implantable pump.

The implant can be comprised of any suitable material, such as a metal (*e.g*., titanium), metal composite, organic material, polymeric, or even ceramic material. The surface of the implant can be any shaped surface, and may have a porous, beaded or meshed ingrowth surface, as can be present in certain implants.

The implant device can be any type of medical implant. The implant devices can include, for example, implants for drug delivery, including drug delivery pumps; orthopedic implants, including spinal implants, implants for osseointegration or bone repair; medical stents, including stents with inherent drug delivery capability; prosthetic implants, including breast implants, muscle implants, and the like; dental implants; ear implants, including cochlear implants and hearing devices; cardiac implants including pacemakers, catheters, etc.; space filling implants; bioelectric implants; neural implants; internal organ implants, including dialysis grafts; defribrillators; monitoring devices; recording devices; stimulators, including deep brain stimulators, nerve stimulators, bladder stimulators, and diaphragm stimulators; implantable identification devices and information chips; artificial organs; drug administering devices; implantable sensors/biosensors; screws, tubes, rods, plates, or artificial joints.

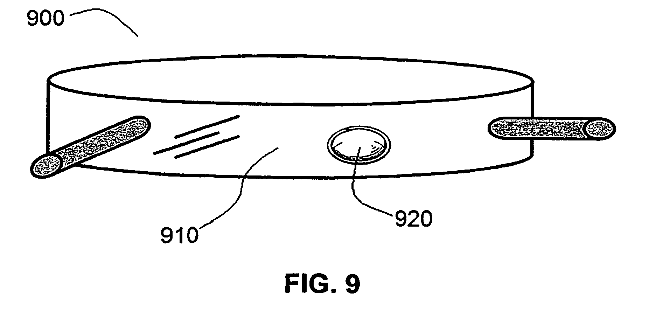

In a further aspect, the implant device can be at least one of a pump, pacemaker, defribrillator, or stimulator, including deep brain stimulators, nerve stimulators, bladder stimulators, and diaphragm stimulators. With reference to Figure 9, an implant device **900** can comprise a first implant device surface **910** that comprises a suction cup composite **920.**

Once the implant device is present in a subject, the suction cup composite can degrade, allowing the bioactive agent to be released in or near the tissue that is adjacent the implant site. If desired, a plurality of suction cup composites can be applied to the implant device. Optionally, the suction cup composite can fall off the contacting surface once inside the subject. In one aspect, the suction cup composite can fall off the contacting surface once the function of the suction cup composite has been completed, for example, the delivery of the bioactive agent to adjacent tissues or fluids of the subject.

Other implant devices that may benefit when used with the disclosed suction cup composites include those with one or more active surfaces, *e.g*., a surface that enhances a connection between a tissue or fluid and the implant device, or a surface that allows for or enhances wound healing. The disclosed suction cup composites can be effective when applied to only a portion of the implant device, allowing for any active surface to remain exposed and functional when the implant device is implanted in a subject.

As discussed above, it can be desirable to deliver a bioactive agent at or near the tissue adjacent an implant site. The bioactive agent can help prevent some of the problems associated with implants, such as infection, or enhance the function of the implant itself. It can also be desirable to avoid pre-manufacturing an implant device with bioactive agent releasing capability, as discussed above. It should be appreciated that the composites, methods, and kits disclosed herein can allow for a point of use application of a suction cup composite onto the surface of an implant device, thus obviating the need to pre-manufacture implant devices having bioactive agent releasing capability.

In one aspect, a suction cup composite can be applied to an implant device surface close to or during the time of use. For example, a suction cup composite can be applied to an implant device by securing the suction cup composite onto the surface of the implant device, substantially close to the time when the implant device is implanted in a subject. In one aspect, the suction cup composite can be applied to an implant device in an operating suite, for example, by a physician or nurse.

The suction cup composite can be secured to the surface of the implant device prior to or after the time when the implant device is implanted in the subject. In one aspect, the implant device comprising the suction cup composite can be implanted into the subject. In a further aspect, the implant device can be implanted into the subject, and then the suction cup composite can be applied to the surface of the implant device. When implanting smaller implants, it may be beneficial to first secure the suction cup composite to the implant device surface before implanting the device in a subject.

In one aspect, the suction cup composite can be secured to the surface of the implant device on the same day (i.e., within 24 hours) of the implant surgery, including, for example, within 23 hours, 20 hours, 15 hours, 10 hours, 5 hours, 3 hours, 2 hours, 1 hour, 30 minutes, 15 minutes, 10 minutes, 5 minutes, 2 minutes, 30 seconds, or during with the implant surgery itself. In certain aspects, as discussed above, the suction cup composite can remain secured to the device for any desired time interval, including without limitation, days, weeks, or longer.

If desired, the suction cup composite itself, with or without an implant device, can be implanted onto or in a tissue or fluid of a subject. In one aspect, the suction cup composite can be implanted onto or in a tissue or fluid that is near or adjacent to an implant site, *i.e.,* a site where an implant device has been implanted, or near or adjacent to a desired implant site.

Typically, before applying the suction cup composite to the implant device, the implant device surface can be cleaned or treated to remove any surface contaminants and to promote good adhesion of the suction cup composite. For example, the suction cup composite and/or the implant device can be sterilized *e.g*. by autoclaving under water steam. However, in some aspects, care may be needed to avoid irreversibility deforming the suction cup composite, or melting the suction cup composite. The suction cup composite or implant device comprising the suction cup composite can then be implanted into the subject using known surgical techniques. In certain aspects, it can be desirable to store the suction cup composites or kits comprising the composites in a sterilized container or package. In one aspect, the kit can comprise a sterilized package of the suction cup composites.

The disclosed methods can be used with any of the disclosed suction cup composites comprising a releasable bioactive agent. In one aspect, the method comprises securing the suction cup composite on a contacting surface of the implant device, or tissue or fluid of a subject. In one aspect, the substantially planar rim can be applied to a contacting surface (*e.g*., a substantially planar contacting surface) of the implant device. The application of the suction cup composite to a contacting surface can be carried out using any known means, including without limitation manual deformation of the cup member, which in various aspects, expels air from within the cavity defined by the cup member. As the air is expelled, the rim of the cup member forms a tight seal with the contacting surface, allowing the cup member to remain adhered to the contact surface for a desired period of time, including extended periods of time, depending on the tightness of the formed seal. Such a seal is achieved in a suction cup like manner. Optionally, an adhesive can be applied to a portion or the entire first surface of the suction cup composite, or to a portion or the entire implant device surface, to aid in securing the suction cup composite to the implant device. Additionally, as discussed above, which can be used with or without an adhesive, the rim of the cup member can be moistened to aid in making a seal to the contacting surface.

The implant device can be implanted in any desired subject. The subject can be a vertebrate, such as a mammal, a fish, a bird, a reptile, or an amphibian. The subject of the herein disclosed methods can be, for example, a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered.

### Example v.1 (Prophetic)

In a first Example, Particles of tobramycin (antibiotic) and particles of polylactide (biocompatible, bioresorbable polymer) are prepared of a suitable size, e.g., about a mean of 500 micron, and blended. The blended mixture of drug and polymer particles are introduced into a twin-screw, melt extruder. The melt extruder is equipped with a rectangular dye such that a molten mixture comprising tobramycin and polylactide passes through the dye and exits the extruder as molten film which then cools and solidifies into a continuous film. The hardened film contains tobramycin dispersed thoughout the polymer matrix. The resultant film is then heat pressed against a circular, convex mold to form a cup member with flashing. The flashing or excess film is then removed by cutting to make a circular cup member. The cup member is packaged and terminally sterilized. Just prior to administration, the cup member is removed from the sterile package and pushed onto a medical device, e.g., a deep brain stimulator. To aid adherence the cup member can be moistened with sterile water or phosphate buffered saline. Once the cup member is held in place to the medical device by suction, the device is placed inside the patient. Subsequently, extended release of tobramycin from the cup member (drug-eluting film) occurs for a desired period to prevent infection.

### vi) Spray Coating Compositions

Also described herein are spray coating compositions that can be applied to an implant device, or to a tissue or fluid of a subject. The compositions described herein allow for controlled-release, extended-release, modified-release, sustained-release, pulsatile-release, delayed-release, or programmed-release of the bioactive agent.

The polymer used with the spray coating compositions can comprise any biocompatible and biodegradable or non-biodegradable polymer. The polymers disclosed herein can be homopolymers or copolymers. The polymers can be block or blocky co- or ter- polymers, random co- or ter- polymers, star polymers, or dendrimers. Any desired molecular weight polymer can be used, depending on the desired properties of the spray coating composition. In certain aspects, if a high strength spray coating composition is desired, then high molecular weight polymers can be used, for example, to meet strength requirements. In other aspects, low or medium molecular weight polymers can be used when, for example, when resorption time of the polymer, rather than material strength is desired.

The molecular weight of the polymer can be selected so as to provide a desired property of the spray coating composition. In certain aspects, the spray coating composition can be provided by forming a solution or dispersion of the polymer in a volatile solvent. In such aspects, the molecular weight should be such to allow a sufficient solution or dispersion to form. The molecular weight should, in certain aspects, also be suitable to allow the polymer to be propelled through an aerosol container by a pressurized propellant. In some aspects, the polymer can be a low-molecular-weight polymer in order to have sufficient solubility in a biologically relevant solvent system. In other aspects, the polymer can be oligomeric in nature. The molecular weight of a polymer is also important from the point of view that molecular weight influences the biodegradation rate of the polymer. For a diffusional mechanism of bioactive agent release, the polymer should remain intact until all of the drug is released from the polymer and then degrade. The drug can also be released from the polymer as the polymer bioerodes. By an appropriate selection of polymeric materials a polymer formulation can be made such that the resulting biodegradable polymer exhibits both diffusional release and biodegradation release properties. Molecular weights can be measured by methods known in the art, including gel permeation chromatography, viscosity, light-scattering, among other methods.

The spray coating composition can be formulated so as to degrade, once the solvent is volatilized, within a desired time interval, once present in a subject. In some aspects, the time interval can be from about less than one day to about 1 month. Longer time intervals can extend to 6 months, including for example, polymer matrices that degrade from about ≥ 0 to about 6 months, or from about 1 to about 6 months. In other aspects, the polymer can degrade in longer time intervals, up to 2 years or longer, including, for example, from about ≥0 to about 2 years, or from about 1 month to about 2 years.

The desired bioactive agent release mechanism can influence the selection of the polymer. A biodegradable polymer can be selected so as to release or allow the release of a bioactive agent therefrom at a desired lapsed time after the spray coating composition has been applied to a surface. For example, the polymer can be selected to release or allow the release of the bioactive agent prior to the bioactive agent beginning to diminish its activity, as the bioactive agent begins to diminish in activity, when the bioactive agent is partially diminished in activity, for example at least 25%, at least 50% or at least 75% diminished, when the bioactive agent is substantially diminished in activity, or when the bioactive agent is completely gone or no longer has activity.

In one aspect, the polymer can be one or more of polyesters, polyhydroxyalkanoates, polyhydroxybutyrates, polydioxanones, polyhydroxyvalerates, polyanhydrides, polyorthoesters, polyphosphazenes, polyphosphates, polyphosphoesters, polydioxanones, polyphosphoesters, polyphosphates, polyphosphonates, polyphosphates, polyhydroxyalkanoates, polycarbonates, polyalkylcarbonates, polyorthocarbonates, polyesteramides, polyamides, polyamines, polypeptides, polyurethanes, polyalkylene alkylates, polyalkylene oxalates, polyalkylene succinates, polyhydroxy fatty acids, polyacetals, polycyanoacrylates, polyketals, polyetheresters, polyethers, polyalkylene glycols, polyalkylene oxides, polyethylene glycols, polyethylene oxides, polypeptides, polysaccharides, or polyvinyl pyrrolidones. Other non-biodegradable but durable polymers include without limitation ethylene-vinyl acetate co-polymer, polytetrafluoroethylene, polypropylene, polyethylene, and the like. Likewise, other suitable non-biodegradable polymers include without limitation silicones and polyurethanes.

In a further aspect, the polymer can be a poly(lactide), a poly(glycolide), a poly(lactide-co-glycolide), a poly(caprolactone), a poly(orthoester), a poly(phosphazene), a poly(hydroxybutyrate) or a copolymer containing a poly(hydroxybutarate), a poly(lactide-co-caprolactone), a polycarbonate, a polyesteramide, a polyanhydride, a poly(dioxanone), a poly(alkylene alkylate), a copolymer of polyethylene glycol and a polyorthoester, a biodegradable polyurethane, a poly(amino acid), a polyamide, a polyesteramide, a polyetherester, a polyacetal, a polycyanoacrylate, a poly(oxyethylene)/poly(oxypropylene) copolymer, polyacetals, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations, or blends thereof.

In one aspect, the polymer can be a polyester comprising lactide (L), glycolide (G), caprolactone (CPL) (including their copolymers); block copolymers of polyethylene glycol (PEG) with polyesters comprising L, G, or CPL; block copolymers of PVP with polyesters comprising L, G, CPL; and similar polymers and copolymers comprising other polyesters (such as dioxanone, hydroxyvalerate, orthoester, hydroxybutyrate, modified polyesters such as hexyl-modified polylactides, and so on) as well as polymers and copolymers comprising other biodegradable polymers (such as polyanhydrides, polyorthoesters, polyphosphates, poly carbonates, polyalkylcarbonates, polyesteramides, polyurethanes, polyetheresters, polypeptides, proteins, polysaccharides, modified polysaccharides, starches, chitosan, modified chitosan, albumin, hyaluronic acid, and the like).

In one aspect, the polymeric material could be a viscous terpolymer that is dissolved in the solvent system at a suitable level (1%, 5%, 10%, 20%, 40%, for example). Similarly, the polymeric material could be a hex-modified poly(lactide).

In one aspect, useful biodegradable polymers are those that comprise one or more blocks of hydrophilic or water soluble polymers, including, but not limited to, polyethylene glycol, (PEG), or polyvinyl pyrrolidone (PVP), in combination with one or more blocks another biocompabible or biodegradable polymer that comprises lactide, glycolide, caprolactone, or a combination thereof.

In a still further aspect, useful biodegradable polymers are those that comprise one or more residues of lactic acid, glycolic acid, lactide, glycolide, caprolactone, hydroxybutyrate, hydroxyvalerates, dioxanones, polyethylene glycol (PEG), polyethylene oxide, or a combination thereof. In a still further aspect, useful biodegradable polymers are those that comprise one or more residues of lactide, glycolide, caprolactone, or a combination thereof.

In specific aspects, the biodegradable polymer can comprise one or more lactide residues. The polymer can comprise any lactide residue, including all racemic and stereospecific forms of lactide, including, but not limited to, L-lactide, D-lactide, and D,L-lactide, or a mixture thereof. Useful polymers comprising lactide include, but are not limited to poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide polymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL- polymers. In addition to copolymers comprising glycolide and DL-lactide or L-lactide, copolymers of L-lactide and DL-lactide are commercially available. Homopolymers of lactide or glycolide are also commercially available.

When the biodegradable polymer is poly(lactide-co-glycolide), poly(lactide), or poly(glycolide), the amount of lactide and glycolide in the polymer can vary. In a further aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In a further aspect, the biodegradable polymer can be poly(lactide), 95:5 poly(lactide-co-glycolide) 85:15 poly(lactide-co-glycolide), 75:25 poly(lactide-co-glycolide), 65:35 poly(lactide-co-glycolide), or 50:50 poly(lactide-co-glycolide), where the ratios are mole ratios.

In a further aspect, the polymer can be a poly(caprolactone) or a poly(lactide-co-caprolactone). In one aspect, the polymer can be a poly(lactide-caprolactone), which, in various aspects, can be 95:5 poly(lactide-co-caprolactone), 85:15 poly(lactide-co-caprolactone), 75:25 poly(lactide-co- caprolactone), 65:35 poly(lactide-co- caprolactone), or 50:50 poly(lactide-co- caprolactone), where the ratios are mole ratios.

In one aspect, the polymer can be a low-molecular-weight polylactide or poly(lactide-co-glycolide) or poly(lactide-co-caprolactone) that is capable of being dissolved at a suitable concentration in an appropriate solvent system (for example, 2-5% polymer in ethanol or an ethanolic solvent) which is then used in a aerosol container or pump sprayer as a system for administration and delivery of a suitable bioactive agent.

In a further aspect, the spray coating composition can comprise a terpolymer. In one aspect, the terpolymers are those terpolymers disclosed in U.S. Patent Publication No. 2009/0124535.

It is understood that any combination of the aforementioned biodegradable polymers can be used, including, but not limited to, copolymers thereof, mixtures thereof, or blends thereof. Likewise, it is understood that when a residue of a biodegradable polymer is disclosed, any suitable polymer, copolymer, mixture, or blend, that comprises the disclosed residue, is also considered disclosed. To that end, when multiple residues are individually disclosed (*i.e*., not in combination with another), it is understood that any combination of the individual residues can be used. Further, any of the above polymers can be processed (*e.g*., cross-linked to a desired level, to achieve a desired property). An additional cross-linking agent can be used, and/or radical, cation, or anion cross-linking of the existing polymer can be used.

The polymer can be dissolved or dispersed in a suitable solvent or solvent system. In one aspect, the solvent can be a biocompatible solvent. In such aspects, it can be preferred that even if residual solvent is left after most of the solvent is volatilized, after the composition has been sprayed onto an implant device surface, that the residual solvent would not harm the subject. Examples of suitable solvents include without limitation ethanol, ethyl lactate, propylene carbonate, glycofurol, N methylpyrrolidone, 2 pyrrolidone, propylene glycol, acetone, methyl acetate, ethyl acetate, methyl ethyl ketone, benzyl alcohol, triacetin, dimethylformamide, dimethylsulfoxide, tetrahydrofuran, chloroform, dichloromethane, polyethylene glycol, CFC propellants, non-CFC propellants, or a combination or mixture thereof. Such solvents can comprise water at an acceptable level.

As discussed above, the spray coating composition comprises a bioactive agent. The bioactive agent can be a releasable bioactive agent, *i.e*., a bioactive agent that can be released from the composition, once coated on a surface. In one aspect, the bioactive agent can be dissolved or dispersed in the at least one solvent, and/or in the polymer.

Also disclosed are kits comprising the spray coating compositions. The kit can be comprised one or more disclosed spray coating compositions, in a package. Such a kit may be useful for point of use applications of the spray coating compositions.

Also disclosed are containers comprising the spray coating compositions that can be used to apply the spray coating compositions onto a surface. In one aspect, the container is an aerosol container. An aerosol container is a type of dispensing system which creates an aerosol mist of liquid particles. Such a container can comprise the spray coating compositions as a liquid under pressure. The container can have a valve, which when opened, allows the spray coating composition to be forced out of the container as an aerosol or mist. Typically, an aerosol container can comprise a propellant, which is usually a gas that can expand to drive out the spray coating composition, while some propellant can evaporate inside the container to maintain an even pressure. Once outside the container, the droplets of propellant preferably evaporate rapidly, leaving the spray coating composition suspended as fine particles or droplets.

The propellant, when present, can be any suitable propellant. In one aspect, the propellant can be one or more of volatile hydrocarbons, such as, for example, propane, n-butane and isobutane. In one aspect, the propellant can comprise a CFC or non-CFC propellant. In one aspect, the propellant can be dimethyl ether (DME) or methyl ethyl ether. In a further aspect, the propellant can be a gas. For example, suitable gases included without limitation nitrous oxide and carbon dioxide. In one aspect, the propellant can be those propellants typically used in medical applications. Examples of such include without limitation hydrofluoroalkanes (HFA), such as, for example, HFA 134a (1,1,1,2,-tetrafluoroethane) or HFA 227 (1,1,1,2,3,3,3-heptafluoropropane), or a combination thereof. Methods of making the dispersion systems and aerosol containers herein can be carried out by methods known in the art.

In other aspects, the solvent system can first be presented separately from the bioactive agent / polymer mixture in a two component system. In one aspect, the solvent system can be added to the bioactive agent / polymer mixture at time of use. Following suitable mixing, the dissolved/dispersed system could be sprayed onto a surface, as discussed above.

Also disclosed are implant devices comprising the coating compositions. The term "device" is any formulation or article that is greater than 1 mm in length in at least one dimension of the device. The device can comprise a disclosed coating composition, with or without the solvent. In a further aspect, the device has one dimension that is from 1 mm to 50 mm, 1.2 mm to 45 mm, 1.4 mm to 42 mm, 1.6 mm to 40 mm, 1.8 mm to 38 mm, or 2.0 mm to 36 mm, 5.0 mm to 33 mm, or 10 mm to 30 mm. In a further aspect, the device has one dimension that is greater than 3 cm, even up to or greater than 10 cm, 20 cm, or even 30 cm.

In one aspect, the implant device comprises a disclosed spray coating composition, with or without the solvent, contacting at least a portion of an implant device surface.

The implant device can comprise any shape, such as a rod, a fiber, a cylinder, a bead, a ribbon, a disc, a wafer, a free-formed shaped solid, or a variety of other shaped solids. The device can have any regular or irregular shape and can have any cross section like circular, rectangular, triangular, oval, and the like. In a further aspect, the device comprises a cylindrical shape, such as a typical shape of an implantable pump.

The implant can be comprised of any suitable material, such as a metal (*e.g*., titanium), metal composite, organic material, polymeric, or even ceramic material. The surface of the implant can be any shaped surface, and may have a porous, beaded or meshed ingrowth surface, as can be present in certain implants.

The implant device can be any type of medical implant. The implant devices can include, for example, implants for drug delivery, including drug delivery pumps; orthopedic implants, including spinal implants, implants for osseointegration or bone repair; medical stents, including stents with inherent drug delivery capability; prosthetic implants, including breast implants, muscle implants, and the like; dental implants; ear implants, including cochlear implants and hearing devices; cardiac implants including pacemakers, catheters, etc.; space filling implants; bioelectric implants; neural implants; internal organ implants, including dialysis grafts; defribrillators; monitoring devices; recording devices; stimulators, including deep brain stimulators, nerve stimulators, bladder stimulators, and diaphragm stimulators; implantable identification devices and information chips; artificial organs; drug administering devices; implantable sensors/biosensors; screws, tubes, rods, plates, or artificial joints.

In a further aspect, the implant device can be at least one of a pump, pacemaker, defribrillator, or stimulator, including deep brain stimulators, nerve stimulators, bladder stimulators, and diaphragm stimulators.

Once the implant device is present in a subject, the spray coating composition can degrade, allowing the bioactive agent to be released in or near the tissue that is adjacent the implant site. If desired, a plurality of spray coating compositions can be applied to the implant device. Optionally, the spray coating composition can fall off the contacting surface once inside the subject. In one aspect, the spray coating composition can fall off the contacting surface once the function of the spray coating composition has been completed, for example, the delivery of the bioactive agent to adjacent tissues or fluids of the subject.

Other implant devices that may benefit when used with the disclosed spray coating compositions include those with one or more active surfaces, e.g., a surface that enhances a connection between a tissue or fluid and the implant device, or a surface that allows for or enhances wound healing. The disclosed spray coating compositions can be effective when applied to only a portion of the implant device, allowing for any active surface to remain exposed and functional when the implant device is implanted in a subject.

As discussed above, it can be desirable to deliver a bioactive agent at or near the tissue adjacent an implant site. The bioactive agent can help prevent some of the problems associated with implants, such as infection, or enhance the function of the implant itself. It can also be desirable to avoid pre-manufacturing an implant device with bioactive agent releasing capability, as discussed above. It should be appreciated that the compositions, methods, systems and kits disclosed herein can allow for a point of use application of a spray coating composition onto the surface of an implant device, thus obviating the need to pre-manufacture implant devices having bioactive agent releasing capability.

In one aspect, a spray coating composition can be applied to an implant device surface close to or during the time of use. For example, a spray coating composition can be applied to an implant device by spraying the spray coating composition onto the surface of the implant device, substantially close to the time when the implant device is implanted in a subject. In one aspect, the spray coating composition can be applied to an implant device in an operating suite, for example, by a physician or nurse.

The spray. coating composition can be sprayed onto the surface of the implant device prior to or after the time when the implant device is implanted in the subject. In one aspect, the implant device comprising the spray coating composition can be implanted into the subject. In a further aspect, the implant device can be implanted into the subject, and then the spray coating composition can be sprayed onto the surface of the implant device. When implanting smaller implants, it may be beneficial to first spray the spray coating composition onto the implant device surface before implanting the device in a subject.

In one aspect, the spray coating composition can be sprayed onto the surface of the implant device on the same day (*i.e*., within 24 hours) of the implant surgery, including, for example, within 23 hours, 20 hours, 15 hours, 10 hours, 5 hours, 3 hours, 2 hours, 1 hour, 30 minutes, 15 minutes, 10 minutes, 5 minutes, 2 minutes, 30 seconds, or during with the implant surgery itself.

If desired, the spray coating composition itself, with or without an implant device, can be sprayed onto or in a tissue or fluid of a subject. In one aspect, the spray coating composition can be sprayed onto or in a tissue or fluid that is near or adjacent to an implant site, *i.e.,* a site where an implant device has been implanted, or near or adjacent to a desired implant site. In such aspects, a biocompatible solvent would be preferred.

Typically, before spraying the spray coating composition onto the implant device, the implant device surface can be cleaned or treated to remove any surface contaminants and to promote good adhesion of the spray coating composition. For example, the spray dispensing system (*e.g*., the aerosol container) and/or the implant device can be sterilized. In certain aspects, it can be desirable to store the spray coating compositions or kits comprising the compositions in a sterilized container or package. In one aspect, the kit can comprise a sterilized package of the spray coating compositions, present in a suitable dispersing system.

The disclosed methods can be used with any of the disclosed spray coating compositions comprising a releasable bioactive agent.

The implant device can be implanted in any desired subject. The subject can be a vertebrate, such as a mammal, a fish, a bird, a reptile, or an amphibian. The subject of the herein disclosed methods can be, for example, a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered.

### Bioactive Agents

As discussed above, each of the disclosed implantable composites and compositions comprise a bioactive agent. The bioactive agent can be a releasable bioactive agent, *i.e*., a bioactive agent that can be released from the polymer film. In certain aspects, the bioactive agent can be in or on the polymer film.

Various forms of the bioactive agent can be used, which are capable of being released into adjacent tissues or fluids. A liquid or solid bioactive agent can be incorporated into the composites and compositions described herein. The bioactive agents are at least very slightly water soluble, and preferably moderately water soluble. The bioactive agents can include salts of the active ingredient. As such, the bioactive agents can be acidic, basic, or amphoteric salts. They can be nonionic molecules, polar molecules, or molecular complexes capable of hydrogen bonding. The bioactive agent can be included in the compositions in the form of, for example, an uncharged molecule, a molecular complex, a salt, an ether, an ester, an amide, polymer drug conjugate, or other form to provide the effective biological or physiological activity.

Examples of bioactive agents that incorporated into systems herein include, but are not limited to, peptides, proteins such as hormones, enzymes, antibodies and the like, nucleic acids such as aptamers, iRNA, DNA, RNA, antisense nucleic acid or the like, antisense nucleic acid analogs or the like, low-molecular weight compounds, or high-molecular-weight compounds. Bioactive agents contemplated for use in the disclosed implantable film-based composites include anabolic agents, antacids, anti-asthmatic agents, anti-cholesterolemic and anti-lipid agents, anti-coagulants, anti-convulsants, anti-diarrheals, anti-emetics, anti-infective agents including antibacterial and antimicrobial agents, anti-inflammatory agents, anti-manic agents, antimetabolite agents, anti-nauseants, antineoplastic agents, anti-obesity agents, anti-pyretic and analgesic agents, anti-spasmodic agents, anti-thrombotic agents, anti-tussive agents, anti-uricemic agents, anti-anginal agents, antihistamines, appetite suppressants, biologicals, cerebral dilators, coronary dilators, bronchiodilators, cytotoxic agents, decongestants, diuretics, diagnostic agents, erythropoietic agents, expectorants, gastrointestinal sedatives, hyperglycemic agents, hypnotics, hypoglycemic agents, immunomodulating agents, ion exchange resins, laxatives, mineral supplements, mucolytic agents, neuromuscular drugs, peripheral vasodilators, psychotropics, sedatives, stimulants, thyroid and anti-thyroid agents, tissue growth agents, uterine relaxants, vitamins, or antigenic materials.

Other bioactive agents include androgen inhibitors, polysaccharides, growth factors, hormones, anti-angiogenesis factors, dextromethorphan, dextromethorphan hydrobromide, noscapine, carbetapentane citrate, chlophedianol hydrochloride, chlorpheniramine maleate, phenindamine tartrate, pyrilamine maleate, doxylamine succinate, phenyltoloxamine citrate, phenylephrine hydrochloride, phenylpropanolamine hydrochloride, pseudoephedrine hydrochloride, ephedrine, codeine phosphate, codeine sulfate morphine, mineral supplements, cholestryramine, N-acetylprocainamide, acetaminophen, aspirin, ibuprofen, phenyl propanolamine hydrochloride, caffeine, guaifenesin, aluminum hydroxide, magnesium hydroxide, peptides, polypeptides, proteins, amino acids, hormones, interferons, cytokines, and vaccines.

Representative drugs that can be used as bioactive agents in the systems herein include, but are not limited to, peptide drugs, protein drugs, desensitizing materials, antigens, anti-infective agents such as antibiotics, antimicrobial agents, antiviral, antibacterial, antiparasitic, antifungal substances and combination thereof, antiallergenics, androgenic steroids, decongestants, hypnotics, steroidal anti-inflammatory agents, anticholinergics, sympathomimetics, sedatives, miotics, psychic energizers, tranquilizers, vaccines, estrogens, progestational agents, humoral agents, prostaglandins, analgesics, antispasmodics, antimalarials, antihistamines, cardioactive agents, nonsteroidal anti-inflammatory agents, antiparkinsonian agents, antihypertensive agents, β-adrenergic blocking agents, nutritional agents, and the benzophenanthridine alkaloids. The agent can further be a substance capable of acting as a stimulant, sedative, hypnotic, analgesic, anticonvulsant, and the like.

The systems herein can comprise a large number of bioactive agents either singly or in combination. Other bioactive agents include but are not limited to analgesics such as acetaminophen, acetylsalicylic acid, and the like; anesthetics such as lidocaine, xylocaine, and the like; anorexics such as dexadrine, phendimetrazine tartrate, and the like; antiarthritics such as methylprednisolone, ibuprofen, and the like; antiasthmatics such as terbutaline sulfate, theophylline, ephedrine, and the like; antibiotics such as sulfisoxazole, penicillin G, ampicillin, cephalosporins, amikacin, gentamicin, tetracyclines, chloramphenicol, erythromycin, clindamycin, isoniazid, rifampin, and the like; antifungals such as amphotericin B, nystatin, ketoconazole, and the like; antivirals such as acyclovir, amantadine, and the like; anticancer agents such as cyclophosphamide, methotrexate, etretinate, and the like; anticoagulants such as heparin, warfarin, and the like; anticonvulsants such as phenytoin sodium, diazepam, and the like; antidepressants such as isocarboxazid, amoxapine, and the like;antihistamines such as diphenhydramine HCl, chlorpheniramine maleate, and the like; hormones such as insulin, progestins, estrogens, corticoids, glucocorticoids, androgens, and the like; tranquilizers such as thorazine, diazepam, chlorpromazine HCl, reserpine, chlordiazepoxide HCl, and the like; antispasmodics such as belladonna alkaloids, dicyclomine hydrochloride, and the like; vitamins and minerals such as essential amino acids, calcium, iron, potassium, zinc, vitamin B₁₂, and the like; cardiovascular agents such as prazosin HCl, nitroglycerin, propranolol HCl, hydralazine HCl, pancrelipase, succinic acid dehydrogenase, and the like; peptides and proteins such as LHRH, somatostatin, calcitonin, growth hormone, glucagon-like peptides, growth releasing factor, angiotensin, FSH, EGF, bone morphogenic protein (BMP), erythopoeitin (EPO), interferon, interleukin, collagen, fibrinogen, insulin, Factor VIII, Factor IX, Enbrel^{®}, Rituxam^{®}, Herceptin^{®}, alpha-glucosidase, Cerazyme/Ceredose^{®}, vasopressin, ACTH, human serum albumin, gamma globulin, structural proteins, blood product proteins, complex proteins, enzymes, antibodies, monoclonal antibodies, and the like; prostaglandins; nucleic acids; carbohydrates; fats; narcotics such as morphine, codeine, and the like, psychotherapeutics; anti-malarials, L-dopa, diuretics such as furosemide, spironolactone, and the like; antiulcer drugs such as rantidine HCl, cimetidine HCl, and the like.

The bioactive agent can also be an immunomodulator, including, for example, cytokines, interleukins, interferon, colony stimulating factor, tumor necrosis factor, and the like; allergens such as cat dander, birch pollen, house dust mite, grass pollen, and the like; antigens of bacterial organisms such as *Streptococcus pneumoniae, Haemophilus influenzae, Staphylococcus aureus, Streptococcus pyrogenes, Corynebacterium diphteriae, Listeria monocytogenes, Bacillus anthracis, Clostridium tetani, Clostridium botulinum, Clostridium perfringens. Neisseria meningitides, Neisseria gonorrhoeae, Streptococcus mutans. Pseudomonas aeruginosa, Salmonella typhi, Haemophilus parainfluenzae, Bordetella pertussis, Francisella tularensis, Yersinia pestis, Vibrio cholerae, Legionella pneumophila, Mycobacterium tuberculosis, Mycobacterium leprae, Treponema pallidum, Leptspirosis interrogans, Borrelia burgddorferi, Campylobacter jejuni,* and the like; antigens of such viruses as smallpox, influenza A and B, respiratory synctial, parainfluenza, measles, HIV, SARS, varicella-zoster, herpes simplex 1 and 2, cytomeglavirus, Epstein-Barr, rotavirus, rhinovirus, adenovirus, papillomavirus, poliovirus, mumps, rabies, rubella, coxsackieviruses, equine encephalitis, Japanese encephalitis, yellow fever, Rift Valley fever, lymphocytic choriomeningitis, hepatitis B, and the like; antigens of such fungal, protozoan, and parasitic organisms such as *Cryptococcuc neoformans, Histoplasma capsulatum, Candida albicans, Candida tropicalis, Nocardia asteroids, Rickettsia ricketsii, Rickettsia typhi, Mycoplasma pneumoniae, Chlamyda psittaci, Chlamydia trachomatis, Plasmodium falciparum, Trypanasoma brucei, Entamoeba histolytica, Toxoplasma gondii, Trichomonas vaginalis, Schistosoma mansoni,* and the like. These antigens may be in the form of whole killed organisms, peptides, proteins, glycoproteins, carbohydrates, or combinations thereof.

In a specific aspect, the bioactive agent comprises at least one of an antibiotic, antimicrobial, a growth factor, a growth inhibitor, an immunomodulator, a steroid, or an anti-inflammatory., including without limitation any of those disclosed above.

In a further specific aspect, the bioactive agent comprises an antibiotic. The antibiotic can be, for example, one or more of Amikacin, Gentamicin, Kanamycin, Neomycin, Netilmicin, Streptomycin, Tobramycin, Paromomycin, Ansamycins, Geldanamycin, Herbimycin, Carbacephem, Loracarbef, Carbapenems, Ertapenem, Doripenem, Imipenem/Cilastatin, Meropenem, Cephalosporins (First generation), Cefadroxil, Cefazolin, Cefalotin or Cefalothin, Cefalexin, Cephalosporins (Second generation), Cefaclor, Cefamandole, Cefoxitin, Cefprozil, Cefuroxime, Cephalosporins (Third generation), Cefixime, Cefdinir, Cefditoren, Cefoperazone, Cefotaxime, Cefpodoxime, Ceftazidime, Ceftibuten, Ceftizoxime, Ceftriaxone, Cephalosporins (Fourth generation), Cefepime, Cephalosporins (Fifth generation), Ceftobiprole, Glycopeptides, Teicoplanin, Vancomycin, Macrolides, Azithromycin, Clarithromycin, Dirithromycin, Erythromycin, Roxithromycin, Troleandomycin, Telithromycin, Spectinomycin, Monobactams, Aztreonam, Penicillins, Amoxicillin, Ampicillin, Azlocillin, Carbenicillin, Cloxacillin, Dicloxacillin, Flucloxacillin, Mezlocillin, Meticillin, Nafcillin, Oxacillin, Penicillin, Piperacillin, Ticarcillin, Polypeptides, Bacitracin, Colistin, Polymyxin B, Quinolones, Ciprofloxacin, Enoxacin, Gatifloxacin, Levofloxacin, Lomefloxacin, Moxifloxacin, Norfloxacin, Ofloxacin, Trovafloxacin, Sulfonamides, Mafenide, Prontosil (archaic), Sulfacetamide, Sulfamethizole, Sulfanilimide (archaic), Sulfasalazine, Sulfisoxazole, Trimethoprim, Trimethoprim-Sulfamethoxazole (Co-trimoxazole) (TMP-SMX), Tetracyclines, including Demeclocycline, Doxycycline, Minocycline, Oxytetracycline, Tetracycline, and others; Arsphenamine, Chloramphenicol, Clindamycin, Lincomycin, Ethambutol, Fosfomycin, Fusidic acid, Furazolidone, Isoniazid, Linezolid, Metronidazole, Mupirocin, Nitrofurantoin, Platensimycin, Pyrazinamide, Quinupristin/Dalfopristin, Rifampicin (Rifampin in U.S.), Tinidazole, or a combination thereof. In one aspect, the bioactive agent can be a combination of Rifampicin (Rifampin in U.S.) and Minocycline.

It is contemplated that other components such as, for example, excipients, pharmaceutically carriers or adjuvants, microparticles, and the like, can be combined with the composite, composition or bioactive agent. Thus, in certain aspects, the bioactive agent can be present as a component in a pharmaceutical composition. Pharmaceutical compositions can be conveniently prepared in a desired dosage form, including, for example, a unit dosage form or controlled release dosage form, and prepared by any of the methods well known in the art of pharmacy. In general, pharmaceutical compositions are prepared by uniformly and intimately bringing the bioactive agent into association with a liquid carrier or a finely divided solid carrier, or both. The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen. Other pharmaceutically acceptable carriers or components that can be mixed with the bioactive agent can include, for example, a fatty acid, a sugar, a salt, a water-soluble polymer such as polyethylene glycol, a protein, polysacharride, or carboxmethyl cellulose, a surfactant, a plasticizer, a high- or low-molecular-weight porosigen such as polymer or a salt or sugar, or a hydrophobic low-molecular-weight compound such as cholesterol or a wax.

In certain aspects, the polymer and bioactive agent are combined or admixed to form a blend or admixture. Admixing methods can be performed using techniques known in the art. For example, the polymer and bioactive agent can be dry blended (*i.e*., mixing of particulates of the polymer and the agent) using, for example, a Patterson-Kelley V-blender, or granulated prior to processing.

In one aspect, the processing of the admixture can be performed under conditions such that the agent is intimately mixed or dispersed throughout the polymer film. Alternatively, the processing of the admixture can be performed under conditions such that the agent is localized on or in only a portion or portions of the polymer film. The polymer film can include areas that are rich in bioactive agent, and areas that are not as rich. The admixture can be processed by a variety of techniques, such as, for example, melt extruding, injection molding, compression molding, or roller compacting the admixture into a desired shape or structure.

Other suitable pharmaceutical carriers include without limitation microparticles. The term "microparticle" is used herein to refer generally to a variety of substantially structures having sizes from about 10 nm to 2000 microns (2 millimeters) and includes microcapsule, microsphere, nanoparticle, nanocapsule, nanosphere as well as particles, in general, that are less than about 2000 microns (2 millimeters). The microparticle can contain and effect the release of the bioactive agent from the polymer film.

The microparticle can be comprised of any of those polymers mentioned above or any polymer used in the microparticle art. In general, the above mentioned polymers can be cross-linked to a certain level, which thereby can form a microparticle of the polymer, as is known in the art. When a microparticle is present in the polymer film, the microparticle can be the same or different as the polymer comprising the bulk of the polymer film. The polymer film can comprise any desired amount of microparticles, including, for example, from about 1 weight % to about 95 weight %, including 5, 10, 20, 30, 40, 50, 60, 70, 80, and 90 weight %, relative to the weight of the total polymer film. The microparticle can be combined with the polymer film through known methods.

In one aspect, the disclosed microparticles can have an average or mean particle size of from about 20 microns to about 125 microns. In one embodiment the range of mean particle size is from about 40 microns to about 90 microns. In another embodiment the range of mean particle sizes is from about 50 microns to about 80 microns. Particle size distributions are measured by laser diffraction techniques known to those of skill in the art.

In a further aspect, the bioactive agent can be encapsulated, microencapsulated, or otherwise contained within a microparticle. The microparticle can modulate the release of the bioactive agent. The microparticle can comprise any desired amount of the bioactive agent. For example, the microparticle can comprise 1%, 5%, 10%, 20%, 30%, 40%, 50%, 60%, 70%, 80%, 90%, 95% by weight bioactive agent, relative to the weight of the microparticle, including any range between the disclosed percentages.

The microparticles can be made using methods known in the art, including, for example, those methods disclosed in U.S. Patent Publication No. 2007/0190154, published August 16,2007, and U.S. Patent No. 5,407,609 to Tice et al..

As will be apparent, depending upon processing conditions, the polymer used as a starting material in the admixing step may or may not be the same polymer present in the final implantable composite. For example, the polymer during processing may undergo polymerization or depolymerization reactions, which ultimately can produce a different polymer that was used prior to processing. Thus, the term "polymer" as used herein covers the polymers used as starting materials as well as the final polymer present in the device produced by the methods described herein.

## Claims

1. An implantable composite comprising: a tacky biocompatible polymer film comprising a releasable bioactive agent; and a release liner affixed to the tacky biocompatible polymer film, wherein the tacky biocompatible polymer film comprises an adhering terpolymer.

2. An implantable composite comprising: a biocompatible polymer film having at least a first surface comprising an adhesive on at least a portion thereof, wherein a release liner is affixed to the adhesive; and wherein a releasable bioactive agent is in or on the film.

3. An implantable composite comprising: a biocompatible polymer film having at least a first surface and comprising a releasable bioactive agent; and an adhesive on at least a portion of the first surface; wherein the implantable composite has a length dimension and a width dimension, wherein the length dimension is substantially the same as the width dimension.

4. An implantable composite comprising: a biocompatible polymer film having at least a first surface and comprising a releasable bioactive agent; and an adhesive on at least a portion of the first surface; wherein the implantable composite has a shape that is not rectangular.

5. The implantable composite of claim 3 or 4, wherein the implantable composite comprises a release liner affixed to the adhesive.

6. The implantable composite of claim 1, 2, 4, or 5, wherein the implantable composite has a length dimension and a width dimension, wherein the length dimension is substantially the same as the width dimension.

7. The implantable composite of claim 1, 2, 3, or 5, wherein the implantable composite has a shape that is not rectangular.

8. The implantable composite of any of the preceding claims, wherein the bioactive agent is contained within a microparticle.

9. The implantable composite of any of the preceding claims, wherein the bioactive agent is encapsulated within a microparticle.

10. The implantable composite of any of the preceding claims, wherein the bioactive agent comprises at least one of an antibiotic, antimicrobial, a growth factor, a growth inhibitor, an immunomodulator, a steroid, or an anti-inflammatory.

11. An article comprising a plurality of implantable composites that each comprise a biocompatible polymer film comprising a releasable bioactive agent; and one or more release liners affixed or adhered to each implantable composite.

12. An implant device having a first implant device surface comprising the implantable composite of any of claims 1, 3, 4, or 6-10 on at least a portion of the first implant device surface.

13. The implantable composite of claim 1, wherein the adhering terpolymer comprises poly(lactide-co-caprolactone), poly(glycolide-co-caprolactone), or a combination, mixture, or blend thereof.

## Patentansprüche

1. lmplantierbarer Verbundstoff, der einen klebrigen biokompatiblen Polymerfilm, der ein freisetzbares bioaktives Mittel aufweist, und eine an dem klebrigen biokompatiblen Polymerfilm befestigte Trennschicht aufweist, wobei der klebrige biokompatible Polymerfilm ein haftendes Terpolymer aufweist.

2. Implantierbarer Verbundstoff, der einen biokompatiblen Polymerfilm aufweist, der mindestens eine erste Oberfläche hat, die einen Klebstoff auf mindestens einem Teil davon aufweist, wobei eine Trennschicht an dem Klebstoff befestigt ist; und wobei ein freisetzbares bioaktives Mittel in oder auf dem Film ist.

3. Implantierbarer Verbundstoff, der einen biokompatiblen Polymerfilm, der mindestens eine erste Oberfläche hat und ein freisetzbares bioaktives Mittel aufweist; und einen Klebstoff auf mindestens einem Teil der ersten Oberfläche aufweist; wobei der implantierbare Verbundstoff eine Längendimension und eine Breitendimension hat, wobei die Längendimension im Wesentlichen gleich der Breitendimension ist.

4. Implantierbarer Verbundstoff, der einen biokompatiblen Polymerfilm, der mindestens eine erste Oberfläche hat und ein freisetzbares bioaktives Mittel aufweist; und einen Klebstoff auf mindestens einem Teil der ersten Oberfläche aufweist; wobei der implantierbare Verbundstoff eine Form hat, die nicht rechteckig ist.

5. Implantierbarer Verbundstoff nach Anspruch 3 oder 4, wobei der implantierbare Verbundstoff eine an dem Klebstoff befestigte Trennschicht aufweist.

6. Implantierbarer Verbundstoff nach Anspruch 1, 2, 4, oder 5, wobei der implantierbare Verbundstoff eine Längendimension und eine Breitendimension hat, wobei die Längendimension im Wesentlichen gleich der Breitendimension ist.

7. Implantierbarer Verbundstoff nach Anspruch 1, 2, 3, oder 5, wobei der implantierbare Verbundstoff eine Form hat, die nicht rechteckig ist.

8. lmplantierbarer Verbundstoff nach einem der vorhergehenden Ansprüche, wobei das bioaktive Mittel in einem Mikropartikel enthalten ist.

9. Implantierbarer Verbundstoff nach einem der vorhergehenden Ansprüche, wobei das bioaktive Mittel in einem Mikropartikel verkapselt ist.

10. Implantierbarer Verbundstoff nach einem der vorhergehenden Ansprüche, wobei das bioaktive Mittel mindestens eines von einem Antibiotikum, Antimikrobium, einem Wachstumsfaktor, einem Wachstumshemmer, einem Immunmodulator, einem Steroid, oder einem Entzündungshemmer aufweist.

11. Gegenstand, der eine Vielzahl von implantierbaren Verbundstoffen aufweist, die jeweils einen biokompatiblen Polymerfilm, der ein freisetzbares bioaktives Mittel aufweist, und ein oder mehrere an die jeweiligen implantierbaren Verbundstoffe geklebte oder befestigte Trennschichten aufweisen.

12. Implantatvorrichtung, die eine erste Implantatvorrichtungsoberfläche hat, die den implantierbaren Verbundstoff nach einem der Ansprüche 1, 3, 4, oder 6-10 auf mindestens einem Teil der ersten Implantatvorrichtungsoberfläche aufweist.

13. Implantierbarer Verbundstoff nach Anspruch 1, wobei das haftende Terpolymer Poly(lactid-co-caprolacton), Poly(glycolid-co-caprolacton) oder eine Kombination, Gemisch oder eine Mischung davon aufweist.

## Revendications

1. Composite implantable comprenant : un film polymère biocompatible collant comprenant un agent bioactif libérable, et un revêtement antiadhésif déposé sur le film polymère biocompatible collant, dans lequel le film polymère biocompatible collant comprend un terpolymère adhésif.

2. Composite implantable comprenant : un film polymère biocompatible ayant au moins une première surface comprenant un adhésif sur au moins une partie de celle-ci, dans lequel un revêtement antiadhésif est déposé sur l'adhésif, et dans lequel un agent bioactif libérable se trouve dans ou sur le film.

3. Composite implantable comprenant : un film polymère biocompatible ayant au moins une première surface et comprenant un agent bioactif libérable, et un adhésif sur au moins une partie de la première surface, dans lequel le composite implantable a une dimension de longueur et une dimension de largeur, dans lequel la dimension de longueur est sensiblement identique à la dimension de largeur.

4. Composite implantable comprenant : un film polymère biocompatible ayant au moins une première surface et comprenant un agent bioactif libérable, et un adhésif sur au moins une partie de la première surface, dans lequel le composite implantable présente une forme qui n'est pas rectangulaire.

5. Composite implantable selon la revendication 3 ou 4, dans lequel le composite implantable comprend un revêtement antiadhésif déposé sur l'adhésif.

6. Composite implantable selon la revendication 1, 2, 4 ou 5, dans lequel le composite implantable a une dimension de longueur et une dimension de largeur, dans lequel la dimension de largeur est sensiblement identique à la dimension de largeur.

7. Composite implantable selon la revendication 1, 2, 3 ou 5, dans lequel le composite implantable présente une forme qui n'est pas rectangulaire.

8. Composite implantable selon l'une quelconque des revendications précédentes, dans lequel l'agent bioactif est contenu dans une microparticule.

9. Composite implantable selon l'une quelconque des revendications précédentes, dans lequel l'agent bioactif est encapsulé dans une microparticule.

10. Composite implantable selon l'une quelconque des revendications précédentes, dans lequel l'agent bioactif comprend au moins l'un d'un antibiotique, d'un agent antimicrobien, d'un facteur de croissance, d'un inhibiteur de croissance, d'un immunomodulateur, d'un stéroïde ou d'un anti-inflammatoire.

11. Article comprenant une pluralité de composites implantables qui comprennent chacun un film polymère biocompatible comprenant un agent bioactif libérable, et un ou plusieurs revêtements antiadhésifs déposés ou collés sur chaque composite implantable.

12. Dispositif d'implant ayant une première surface de dispositif d'implant comprenant le composite implantable selon l'une quelconque des revendications 1, 3, 4 ou 6 à 10 sur au moins une partie de la première surface de dispositif d'implant.

13. Composite implantable selon la revendication 1, dans lequel le terpolymère adhésif comprend le poly(lactide-co-caprolactone), le poly(glycolide-co- caprolactone), ou une combinaison, une association ou un mélange de ceux-ci.
